# EUROPEAN PATENT SPECIFICATION

(11) **EP 4 307 916 B1**
(45) Date of publication and mention of the grant of the patent: **19.11.2025**
(21) Application number: 22714090.2
(22) Date of filing: 14.03.2022
(51) Int. Cl.: A23K 20/189, A23L 29/00, C12N 9/28, A21D 8/04, G01N 1/30, C11D 3/386, C11D 3/48, C12N 9/54

(54) **USE OF A COMPOSITION AND METHOD FOR BIOLOGICAL SAMPLE PROCESSING**
VERWENDUNG EINER ZUSAMMENSETZUNG UND VERFAHREN ZUR VERARBEITUNG BIOLOGISCHER PROBEN
UTILISATION D'UNE COMPOSITION ET MÉTHODE DE TRAITEMENT D'ÉCHANTILLONS BIOLOGIQUES

(30) Priority: 15.03.2021 US 202163161320 P; 05.01.2022 US 202263296804 P
(43) Date of publication of application: 24.01.2024
(60) Divisional application: 25208592.3
(73) Proprietor: Gen-Probe Incorporated, San Diego, CA 92121 (US)
(72) Inventor: SHAPIRO, Anne-Laure, San Diego, California 92121 (US); TUGGLE, James T., San Diego, California 92121 (US); OPALSKY, David, San Diego, California 92121 (US); NAIR, Sangeetha Vijaysri, San Diego, California 92121 (US)
(74) Representative: Script Intellectual Property LLP
(86) International application number: PCT/US2022/020251
(87) International publication number: WO 2022/197634

(56) References cited:
- WO-A1-94/02597
- WO-A1-99/27082
- US-A1- 2013 005 029
- US-A1- 2015 344 858

## Description

### CROSS REFERENCE TO RELATED APPLICATIONS

This application claims the benefit of priority to United States Provisional Application No. 63/161,320 filed March 15, 2021, and United States Provisional Application No. 63/296,804 filed January 5, 2022.

### TECHNICAL FIELD

This disclosure relates to the field of biotechnology. The present invention concerns the use of a composition and a method for processing biological samples, for example in preparation for use in subsequent analytical procedures.

### INTRODUCTION AND SUMMARY

A biological sample from a subject may be prepared using a variety of methods such that the sample is suitable for storage, processing, and analysis. Throughout sample storage and for as long as needed during sample processing, target analytes in the sample must be preserved while any potentially infectious agent in the sample must be inactivated. Sample transport media are commonly used to stabilize nucleic acids for further analysis during the time after collection from the sample source. Sample transport media can also serve to disrupt the ability of infectious pathogens to replicate or infect a host.

Samples containing solid or viscous matter (e.g., mucus; clots) can be particularly challenging. For example, such samples can complicate specimen transfer steps, including those performed by automated platforms, that are needed for efficiently processing and analyzing the sample. In some instances, such samples can form a precipitate during storage at cold temperatures, e.g., 4°C, -20°C, and -70°C, which can complicate subsequent sample pipetting and loading onto automated instruments. Reducing viscosity and/or dissolving or otherwise liquefying such samples can reduce or eliminate such difficulties.

Thus, there remains a need for a simple, efficient, and fast method to prepare samples for storage, processing, and analysis. Improved sample transport media could meet this need or provide other benefits.

The present invention is defined in the appended claims.

### DETAILED DESCRIPTION

### A. Overview

Certain types of biological samples, such as those containing cells, tissue, cell debris, and/or extracellular biopolymers, can be difficult to analyze using nucleic acid amplification and/or detection procedures. As noted above, samples comprising viscous or particulate matter (e.g., mucus; clots) can be particularly problematic in liquid handling steps. Accordingly, it is often desirable to process such samples prior to downstream analytical steps, e.g., by degrading and/or solubilizing non-nucleic acid sample components. As described in detail below, it has been found that certain compositions can facilitate efficient isolation of nucleic acids by reducing viscosity or otherwise rendering samples more amenable to liquid handling procedures such as aspiration and dispensation. The degree of degradation and/or solubilization of non-nucleic acid components, while not necessarily absolute, can nonetheless be sufficient for improvements such as reduced clogging/clotting, reduced pressure levels, and more consistent results during liquid handling and downstream analytical steps such as nucleic acid amplification or other forms of detection or measurement.

### B. Definitions

"Biological sample" includes any specimen that may contain a target material (e.g., nucleic acid, virus, or other biomolecule), such as any tissue or material derived from or containing a living or dead organism or that may contain target material derived therefrom, including, e.g., peripheral blood, plasma, serum, lymph node, gastrointestinal tissue, cerebrospinal fluid, protein, mucin, sputum, mucus, mucoidal secretions, nasal mucus, pulmonary secretions, stool, urine, semen, vaginal secretion, saliva, biopsy material, other body fluids or materials, soil, sludge, or microbial growth such as a bacterial, archaeal, fungal, or protist colony or biofilm. The biological sample may be treated to physically or mechanically disrupt tissue or cell structure, thus releasing intracellular components into a solution or suspension, and/or combined with a buffer or other components. Also, biological samples include crude samples as discussed below and processed samples, such as those obtained from passing samples over or through a filtering device, or following centrifugation, or by adherence to a medium, matrix, or support.

A "crude sample" is a biological sample obtained directly from a living or dead organism, food, the environment, or the like, in contrast to extracts, lysates, filtrates, or eluates. Merely dissolving, suspending, or otherwise mixing crude material in or with a liquid does not convert it to a processed sample.

A liquid is "aqueous" if it is water, a mixture of water and a liquid miscible with water where the water is present in more than a trace amount, or a solution in which the solvent is any of the foregoing. In some embodiments, an aqueous liquid is at least 50% water by weight.

"Particulate matter" includes any material, excluding whole cells and cellular debris, of sufficient solidity to partially or completely obstruct the aperture of a pipet or micropipette tip. A solute dissolved in a solution is not particulate.

A "suspension" is a liquid containing undissolved solid or particulate matter.

"Aliquot" is used simply to denote some or all of a sample. First and second portions of an aliquot may or may not represent the entire aliquot.

A "viscous polymer" is a polymer that is dissolved in sufficient quantity to detectably increase the viscosity of a liquid upon dissolution. In aqueous or polar liquids, viscous polymers can comprise a plurality of hydrogen bond donors and acceptors, such as hydroxyls, amines, oxos (carbonyl oxygens), etc., such that polymer molecules increase viscosity by exerting sufficient intermolecular attractive forces on a plurality of other polymer molecules and solvent molecules. Polysaccharides, polypeptides, nucleic acids, and various synthetic polymers can all be viscous polymers. Any suitable approach for measuring viscosity may be used. Exemplary instruments for determining viscosity include a capillary viscometer, Zahn cup, vibrational viscometer, and rotational viscometer.

A "polysaccharide" includes any linear or branched chain of sugars (including but not limited to amino sugars and sugar alcohols as well as true carbohydrates), which may or may not be conjugated (e.g., to a lipid or polypeptide) or derivatized (e.g., phosphorylated, acetylated, alkylated, or the like). Polysaccharide chains may use glycosidic or other bonds (e.g., phosphodiesters in certain polysaccharides such as poly-N-acetylmannosamine phosphate).

"Lipids" include fatty acids, triglycerides, phospholipids, sterols, steroids, waxes, and oils.

"Food" includes any material intended or suitable for consumption, including solids, suspensions, emulsions, and gels (thus including gelatin, milk, ice cream, fruit smoothies, emulsified cheese dips, fruit puree, nut butter, processed and/or textured protein, and the like as well as bread, fruit, vegetables, meat, etc., but not including clear, monodisperse solutions or water).

"Nucleic acid" refers to a multimeric compound comprising two or more covalently bonded nucleosides or nucleoside analogs having nitrogenous heterocyclic bases, or base analogs, where the nucleosides are linked together by phosphodiester bonds or other linkages to form a polynucleotide. Nucleic acids include RNA, DNA, and combinations and analogs thereof such as "peptide nucleic acids" or PNAs (see, e.g., WO 95/32305) and "locked nucleic acids" (LNA), in which one or more nucleotide monomers have a bicyclic furanose unit locked in an RNA mimicking sugar conformation (see, e.g., Vester et al., Biochemistry 43:13233-41, 2004). A nucleic acid "backbone" may be made up of a variety of linkages, including one or more of sugar-phosphodiester linkages, peptide-nucleic acid bonds as in PNA, phosphorothioate linkages, methylphosphonate linkages, or combinations thereof. Sugar moieties of the nucleic acid may be either ribose or deoxyribose, or similar compounds having known substitutions such as, for example, 2'-methoxy substitutions and 2'-halide substitutions (e.g., 2'-F). Nitrogenous bases may be conventional bases (A, G, C, T, U), analogs thereof (e.g., inosine, 5-methylisocytosine, isoguanine; see, e.g., The Biochemistry of the Nucleic Acids 5-36, Adams et al., ed., 11th ed., 1992; Abraham et al., 2007, BioTechniques 43: 617-24), which include derivatives of purine or pyrimidine bases (e.g., N⁴-methyl deoxygaunosine, deaza- or aza-purines, deaza- or aza-pyrimidines, pyrimidine bases having substituent groups at the 5 or 6 position, purine bases having an altered or replacement substituent at the 2, 6 and/or 8 position, etc., (see generally U.S. Pat. Nos. 5,378,825, 6,949,367 and International Patent Application Pub. No. WO 93/13121), and/or "abasic" residues (see. e.g., U.S. Pat. No. 5,585,481. The term "polynucleotide" as used herein denotes a nucleic acid chain. A "nucleotide" is a subunit of a nucleic acid having a phosphate group, a 5-carbon sugar, and a nitrogenous base. The term also includes analogs of such subunits, such as a methoxy group at the 2' position of the ribose (also referred to herein as "2'-O-Me" or "2'-methoxy").

A "target" material (such as a target nucleic acid; other embodiments of target materials include cells, viruses, and other biomolecules) as used herein is a material to be detected or quantified. Target nucleic acids may be DNA or RNA as described herein, and may be either single-stranded or double-stranded. The target nucleic acid may include other sequences besides the target sequence, which may not be detected or quantified.

The interchangeable terms "oligomer," "oligo," and "oligonucleotide" refer to a nucleic acid having generally less than 1,000 nucleotide (nt) residues, including polymers in a range having a lower limit of about 5 nt residues and an upper limit of about 500 to 900 nt residues. In some embodiments, oligonucleotides are in a size range having a lower limit of about 12 to 15 nt and an upper limit of about 50 to 600 nt, and other embodiments are in a range having a lower limit of about 15 to 20 nt and an upper limit of about 22 to 100 nt. An oligonucleotide may serve one or more of various different functions, e.g., as a primer and/or promoter, detection probe, capture oligomer, etc.

"Amplifying" or "amplification" refers to any known procedure for obtaining multiple copies of a target nucleic acid sequence or its complement or fragments thereof. The multiple copies may be referred to as amplicons or amplification products, which can be double-stranded or single-stranded and can include DNA, RNA, or both. Amplification of "fragments" refers to production of an amplified nucleic acid that contains less than the complete target nucleic acid or its complement, e.g., produced by using an amplification oligonucleotide that hybridizes to, and initiates polymerization from, an internal position of the target nucleic acid. Known amplification methods include, for example, replicase-mediated amplification, polymerase chain reaction (PCR), ligase chain reaction (LCR), strand-displacement amplification (SDA), helicase-dependent amplification, and transcription-mediated or transcription-associated amplification. Replicase-mediated amplification uses self-replicating RNA molecules, and a replicase such as QB-replicase (see. e.g., U.S. Pat. No. 4,786,600). PCR amplification uses a DNA polymerase, pairs of primers, and thermal cycling to synthesize multiple copies of two complementary strands of dsDNA or from a cDNA (see. e.g., U.S. Pat. Nos. 4,683,195; 4,683,202; and 4,800,159; each incorporated by reference herein). LCR amplification uses four or more different oligonucleotides to amplify a target and its complementary strand by using multiple cycles of hybridization, ligation, and denaturation (see. e.g., U.S. Pat. Nos. 5,427,930 and 5,516,663). SDA uses a primer that contains a recognition site for a restriction endonuclease and an endonuclease that nicks one strand of a hemimodified DNA duplex that includes the target sequence, whereby amplification occurs in a series of primer extension and strand displacement steps (see, e.g., U.S. Pat. Nos. 5,422,252; 5,547,861; and 5,648,211). Helicase-dependent amplification uses a helicase to separate the two strands of a DNA duplex generating single-stranded templates, followed by hybridization of sequence-specific primers hybridize to the templates and extension by DNA polymerase to amplify the target sequence (see, e.g., U.S. Pat. No. 7,282,328). Amplification may be linear or exponential.

"Detection probe," "detection oligonucleotide," "probe oligomer," and "detection probe oligomer" are used interchangeably to refer to a nucleic acid oligomer that hybridizes specifically to a target sequence in a nucleic acid, or in an amplified nucleic acid, under conditions that promote hybridization to allow detection of the target sequence or amplified nucleic acid. Detection may either be direct (e.g., a probe hybridized directly to its target sequence) or indirect (e.g., a probe linked to its target via an intermediate molecular structure). Examples include invasive probes and primary probes, discussed below. Detection probes may be DNA, RNA, analogs thereof or combinations thereof (e.g., DNA/RNA chimerics) and they may be labeled or unlabeled. A detection probe's "target sequence" generally refers to a smaller nucleic acid sequence region within a larger nucleic acid sequence that hybridizes specifically to at least a portion of a probe oligomer by base pairing. A detection probe may comprise target-specific sequences and other sequences that contribute to the three-dimensional conformation of the probe (see. e.g., U.S. Pat. Nos. 5,118,801; 5,312,728; 6,849,412; 6,835,542; 6,534,274; and 6,361,945; and US Patent Application Pub. No. 20060068417).

As used herein, a "label" refers to a moiety or compound joined directly or indirectly to a probe that is detected or leads to a detectable signal. Direct labeling can occur through bonds or interactions that link the label to the probe, including covalent bonds or non-covalent interactions, e.g., hydrogen bonds, hydrophobic and ionic interactions, or formation of chelates or coordination complexes. Indirect labeling can occur through use of a bridging moiety or "linker" such as a binding pair member, an antibody or additional oligomer, which is either directly or indirectly labeled, and which may amplify the detectable signal. Labels include any detectable moiety, such as a radionuclide, ligand (e.g., biotin, avidin), enzyme or enzyme substrate, reactive group, or chromophore (e.g., dye, particle, or bead that imparts detectable color), luminescent compound (e.g., bioluminescent, phosphorescent, or chemiluminescent labels), or fluorophore. Labels may be detectable in a homogeneous assay in which bound labeled probe in a mixture exhibits a detectable change different from that of an unbound labeled probe, e.g., instability or differential degradation properties.

"Capture probe," "capture oligonucleotide," "capture oligomer," "target capture oligomer," and "capture probe oligomer" are used interchangeably to refer to a nucleic acid oligomer that specifically hybridizes to a target sequence in a target nucleic acid by standard base pairing and joins to a binding partner on an immobilized probe to capture the target nucleic acid to a support. One example of a capture oligomer includes two binding regions: a sequence-binding region (e.g., target-specific portion) and an immobilized probe-binding region, usually on the same oligomer, although the two regions may be present on two different oligomers joined together by one or more linkers. Another embodiment of a capture oligomer uses a target-sequence binding region that includes random or non-random poly-GU, poly-GT, or poly-U sequences to bind non-specifically to a target nucleic acid and link it to an immobilized probe on a support.

"Separating" or "purifying" means that one or more components of a sample are removed or separated from other sample components. Sample components include target material, e.g., target nucleic acids, are usually in an aqueous solution phase, which may also include suspended material, cellular fragments, proteins, carbohydrates, lipids, and other nucleic acids. "Separating" or "purifying" does not connote any particular degree of purification. In some embodiments, separating or purifying reduces the amount or concentration of other sample components by at least 70%, or at least 80%, or at least 95%. In some embodiments, separating or purifying renders the target material at least 70%, or at least 80%, or at least 95% pure.

### C. Compositions for Sample Processing

A composition for sample processing may comprise a degradative enzyme, a detergent, and at least one additive, such as a buffer and/or a chelator. The composition used according to the present invention comprises at least one protease and an anionic detergent, and optionally, a buffer and/or a divalent cation chelator, for collection of a biological sample for diagnostic testing,

### 1. Degradative Enzymes

In some embodiments, a degradative enzyme may be wild-type, chemically modified, or engineered. Enzymes may differ in some engineered way from a related wild-type sequence by way of amino acid substitution. For example, a nucleic acid substitution may give rise to an enzyme variant that is different in its specific activity, thermostability, or pH optimum. In some embodiments, an enzyme variant may comprise an amino acid sequence with at least 80%, 85%, 90%, 95%, 96%, 97%, 98%, or 99% sequence identity to a wild-type enzyme.

According to the present invention, the enzyme used is a protease.

In some embodiments, the composition comprises an enzyme concentration of about 0-0.5% w/v, 0.25-0.75% w/v, 0.5-1.0% w/v, 0.75-1.25% w/v, 1.0-1.5% w/v, 1.25-1.75% w/v, 1.5-2.0% w/v, 1.75-2.25% w/v, 2-2.5% w/v, 2.25-2.75% w/v, 2.5-3.0% w/v, 2.75-3.25% w/v, 3-3.5% w/v, 3.25-3.75% w/v, 3.5-4.0% w/v, 3.75-4.25% w/v, 4.0-4.5% w/v, 4.25-4.75% w/v, 4.5-5.0% w/v, 4.75-5.25% w/v, 5.0-5.5% w/v, 5.25-5.75% w/v, 5.5-6.0% w/v, 5.75-6.25% w/v, 6-6.5% w/v, 6.25-6.75% w/v, 6.5-7.0% w/v, or more. In some embodiments, the enzyme concentration is about 1-4% w/v. In some embodiments, the enzyme concentration is about 3% w/v. In some embodiments, the enzyme concentration is about 1-10% w/v, about 3-6% w/v, or about 6% w/v.

In some embodiments, the enzyme may be present at a concentration of less than about 20% by weight of the composition (i.e., w/w). In some embodiments, the enzyme is present at about 0.5-10%, 2-6%, 0.5-1.5%, 1-2%, 1.5-2.5%, 2-3%, 2.5-3.5%, 3-4%, 3.5-4.5%, 4-5%, 4.5-5.5%, 5-6%, 5.5-6.5%, 6-7%, 6.5-7.5%, 7-8%, 7.5-8.5%, 8-9%, 8.5-9.5%, 9-10%, 9.5-10.5%, 10-11%, 10.5-11.5%, 11-12%, 11.5-12.5%, 12-13%, 12.5-13.5%, 13-14%, 13.5-14.5%, 14-15%, 14.5-15.5%, 15-16%, 15.5-16.5%, 16-17%, 16.5-17.5%, 17-8%, 17.5-18.5%, 18-9%, 18.5-19.5%, or 19-20% by weight of the composition. In some embodiments, the enzyme may be present at about 1-4% by weight of the composition. In some embodiments, the enzyme may be present at about 3% by weight of the composition.

In some embodiments, the composition comprises an enzyme (e.g., protease) concentration of about 0-16 anson units per gram (AnsonU/g). In some embodiments, the enzyme concentration is about 0.16 AnsonU/g, about 0.32 AnsonU/g, about 0.48 AnsonU/g, about 0.72 AnsonU/g, about 0.96 AnsonU/g, about 1.6 AnsonU/g, about 3.2 AnsonU/g, about 4.8 AnsonU/g, about 7.2 AnsonU/g, or about 9.6 AnsonU/g. Protease

Suitable proteases include proteases of animal, vegetable, or microbial origin. Wild-type, chemically modified, and protein engineered mutants are included.

Examples of proteases include serine proteases, neutral proteases, threonine proteases, cysteine proteases, aspartate proteases, glutamic acid proteases, metallo proteases, and alkaline proteases.

Serine protease is an enzyme that catalyzes the hydrolysis of peptide bonds and includes an essential serine residue at the active site. In some embodiments, the serine protease is porcine trypsin, bovine trypsin, or Fusarium protease. In some embodiments, the protease is derived from Aspergillus, Rhizopus, *Bacillus alcalophilus, B. cereus, B. vulgatus, B. myocoide,* or Nocardiopsis N. natto.

Neutral protease is an enzyme that catalyzes the hydrolysis of peptide bonds in a neutral, weakly acidic, or weakly alkaline environment. Neutral proteases have optimal proteolytic activity in a neutral pH range of about 6 to about 8, and may be derived from bacterial, fungal, yeast, plant, or animal sources. Examples of neutral proteases include aspartate and metalloproteases. In some embodiments, the metalloprotease is Neutrase^{®} (produced by submerged fermentation of a strain of Bacillus subtilis).

Alkaline protease is catalytically active in a pH that ranges from neutral to alkaline. Examples of alkaline proteases include RP-II proteases and subtilisins. In some embodiments, the protease is derived from Bacillus, Nocardiopsis spe, or *Nocardiopsis dassonvillei.* In some embodiments, the protease is a subtilisin Novo, subtilis Carlsberg, subtilisin 309, subtilisin 147 or subtilisin 168. In some embodiments, the protease is a wild-type RP-II protease or variants thereof, or wild-type subtilisin or variants thereof. In some embodiments, the subtilisin variant comprises substitutions in one or more of the following positions relative to a wild-type subtilisin amino acid sequence: 27, 36, 57, 76, 87, 97, 101, 104, 120, 123, 167, 170, 194, 206, 218, 222, 224, 235 and 274. For an exemplary wild-type subtilisin sequence, see US 6,605,458 B1 at Fig. 1 (BLSAVI sequence, SEQ ID NO: 10 of US 6,605,458 B1).

In some embodiments, a commercially available protease may be used, such as Alcalase^{™}, Duralase^{™}, Esperase^{™}, FN2^{™}, FN3^{™} Kannase^{™}, Maxacal^{™}, Maxapem^{™}, Maxatase^{™}, Primase^{™}, Properase^{™}, Purafect OxP^{™}, Purafect^{™}, and Savinase^{®}. In some embodiments, the protease is Savinase^{®}. In some embodiments, the protease is a subtilisin from *Bacillus lentus* (the species *Bacillus lentus* is also known as *Lederbergia lentus*). Savinase^{®} is an example of a commercially available subtilisin from *Bacillus lentus.*

Suitable conventional fermented commercial proteases may include, for example, Alcalase^{®} (produced by submerged fermentation of a strain of Bacillus licheniformis), Durazyme^{®} (a protein-engineered variant of Savinase^{®}), Esperase^{®} (produced by submerged fermentation of an alkalophilic species of Bacillus), Rennilase^{®} (produced by submerged fermentation of a non-pathogenic strain of Mucor miehei), and Savinase^{®} (produced by submerged fermentation of a genetically modified strain of Bacillus).

In some embodiments, the protease comprises subtilisin A, Alcalase^{®}, Savinase^{®}, Esperase^{®}, Neutrase^{®}, rTrypsin^{®}, or any combination thereof.

In some embodiments, the composition comprises a protease concentration of about 0-0.5% w/v, 0.25-0.75% w/v, 0.5-1.0% w/v, 0.75-1.25% w/v, 1.0-1.5% w/v, 1.25-1.75% w/v, 1.5-2.0% w/v, 1.75-2.25% w/v, 2-2.5% w/v, 2.25-2.75% w/v, 2.5-3.0% w/v, 2.75-3.25% w/v, 3-3.5% w/v, 3.25-3.75% w/v, 3.5-4.0% w/v, 3.75-4.25% w/v, 4.0-4.5% w/v, 4.25-4.75% w/v, 4.5-5.0% w/v, 4.75-5.25% w/v, 5.0-5.5% w/v, 5.25-5.75% w/v, 5.5-6.0% w/v, 5.75-6.25% w/v, 6-6.5% w/v, 6.25-6.75% w/v, 6.5-7.0% w/v, or more. In some embodiments, the protease concentration is about 1-4% w/v. In some embodiments, the protease concentration is about 3% w/v. In some embodiments, the protease concentration is about 1% w/v to 10% w/v, about 3% w/v to 6% w/v, or about 6% w/v.

In some embodiments, the protease may be present at a concentration of less than about 10% by weight of the composition (i.e., w/w). In some embodiments, the protease is present at about 0.5-10%, 2-6%, 0.5-1.5%, 1-2%, 1.5-2.5%, 2-3%, 2.5-3.5%, 3-4%, 3.5-4.5%, 4-5%, 4.5-5.5%, 5-6%, 5.5-6.5%, 6-7%, 6.5-7.5%, 7-8%, 7.5-8.5%, 8-9%, or 8.5-9.5% by weight of the composition. In some embodiments, the protease may be present at about 1-4% by weight of the composition. In some embodiments, the protease may be present at about 3% by weight of the composition. In some embodiments, the protease may be present at about 1% to 10% by weight of the composition, about 3% to 6% by weight of the composition, or about 6% by weight of the composition.

In some embodiments, the composition comprises a protease concentration of about 0-0.5% v/v, 0.25-0.75% v/v, 0.5-1.0% v/v, 0.75-1.25% v/v, 1.0-1.5% v/v, 1.25-1.75% v/v, 1.5-2.0% v/v, 1.75-2.25% v/v, 2-2.5% v/v, 2.25-2.75% v/v, 2.5-3.0% v/v, 2.75-3.25% v/v, 3-3.5% v/v, 3.25-3.75% v/v, 3.5-4.0% v/v, 3.75-4.25% v/v, 4.0-4.5% v/v, 4.25-4.75% v/v, 4.5-5.0% v/v, 4.75-5.25% v/v, 5.0-5.5% v/v, 5.25-5.75% v/v, 5.5-6.0% v/v, 5.75-6.25% v/v, 6-6.5% v/v, 6.25-6.75% v/v, 6.5-7.0% v/v, or more. In some embodiments, the protease concentration is about 1-4% v/v. In some embodiments, the protease concentration is about 2.1-3% v/v. In some embodiments, the protease concentration is about 1 % v/v, 2% v/v, 2.1 % v/v, 2.3 % v/v, 3 % v/v, or 4 % v/v.

### i. Combinations of Proteases

The combination of proteases in a composition may provide synergistic advantages. In some embodiments, a composition may comprise RP-II and subtilisin of the subtilase group I-S2 (Siezen et al., Protein Science, 6: 501-523 91997) or high alkaline subtilisins. Examples of I-S2 proteases include ESPERASE^{®}, MAXACAL^{®}, Savinase^{®}, subtilisin 147, subtilisin 309, subtilisin PB92, and alkaline elastase YaB. In some embodiments, the I-S2 protease is subtilisin from *Bacillus lentus* (the species *Bacillus lentus* is also known as *Lederbergia lentus*).

The combination of proteases may provide an advantage for use with detergents. In some embodiments, a composition may comprise BLC and JA96 and variants thereof with Savinase^{™} and variants thereof, e.g., Duralase^{™}, FN2^{™}, FN3^{™}, Kannase^{™}, Maxacal^{™}, Maxapem^{™}, Maxatase^{™}, Properase^{™}, Purafect OxP^{™}, and Purafect^{™}.

### 2. Detergents

A detergent may be used to lyse cells containing target material and/or to solubilize matter that is otherwise insoluble or poorly soluble in aqueous liquids. Target materials are discussed in Section B above. The detergent may comprise a concentration that is appropriate for cell lysis and desired the catalytic efficiencies of enzymes in the composition. The detergent may be used in a concentrated or diluted form. A detergent may be anionic, non-ionic, or zwitterionic. In some embodiments, the detergent comprises an alkyl sulfate, such as a dodecyl sulfate (also known as a lauryl sulfate). In some embodiments, the detergent comprises a sodium salt and/or a lithium salt. Without wishing to be bound by any particular theory, the combination of an alkyl sulfate detergent with other components described herein (e.g., two or more degradative enzymes) can beneficially reduce problems with sample aspiration due to clots while also maintaining nucleic acids in a form suitable for subsequent amplification and/or detection.

A detergent may comprise a surfactant that is non-ionic, anionic, amphoteric, or cationic, or any combination thereof. Examples of nonionic surfactants include alkanolamides, amine oxides, block polymers, ethoxylated primary and secondary alcohols, ethoxylated alkyphenols, ethoxylated fatty esters, sorbitan derivatives, glycerol esters, propoxylated and ethoxylated fatty acids, alcohols, and alkyl phenols, glycol esters, polymeric polysaccharides, sulfates and sulfonates of ethoxylated alkylphenols, and polymeric surfactants. Examples of anionic surfactants include ethoxylated amines or amides, sulfosuccinates and derivatives, sulfates of ethoxylated alcohols, sulfates of alcohols, sulfonates and sulfonic acid derivatives, phosphate esters, and polymeric surfactants. Examples of amphoteric surfactants include betaine derivatives. Examples of cationic surfactants include amine surfactants.

In some embodiments, the detergent comprises a lithium salt, a lauryl sulfate, or lithium lauryl sulfate.

In some embodiments, the composition comprises a detergent concentration of about 0-0.5% w/v, 0.25-0.75% w/v, 0.5-1.0% w/v, 0.75-1.25% w/v, 1.0-1.5% w/v, 1.25-1.75% w/v, 1.5-2.0% w/v, 1.75-2.25% w/v, 2-2.5% w/v, 2.25-2.75% w/v, 2.5-3.0% w/v, 2.75-3.25% w/v, 3-3.5% w/v, 3.25-3.75% w/v, 3.5-4.0% w/v, 3.75-4.25% w/v, 4.0-4.5% w/v, 4.25-4.75% w/v, 4.5-5.0% w/v, 4.75-5.25% w/v, 5.0-5.5% w/v, 5.25-5.75% w/v, 5.5-6.0% w/v, 5.75-6.25% w/v, 6-6.5% w/v, 6.25-6.75% w/v, 6.5-7.0% w/v, 6.75-7.25% w/v, 7.0-7.5% w/v, 7.25-7.75% w/v, 7.5-8.0% w/v, 7.75-8.25% w/v, 8.0-8.5% w/v, 8.25-8.75% w/v, 8.5-9.0% w/v, 8.75-9.25% w/v, 9.0-9.5% w/v, 9.25-9.75% w/v, 9.5-10.0% w/v, 9.75-10.25% w/v, 10.0-10.5% w/v, 10.25-10.75% w/v, 10.5-11.0% w/v, 10.75-11.25% w/v, 11.0-11.5% w/v, or more. In some embodiments, the detergent concentration is 2.75-3.25% w/v. In any of the foregoing embodiments, the detergent may comprise a lithium salt. In any of the foregoing embodiments, the detergent may comprise a lauryl sulfate. In any of the foregoing embodiments, the detergent may comprise lithium lauryl sulfate.

In some embodiments, the detergent may be present at a concentration of less than about 10% by weight of the composition (i.e., w/w). In some embodiments, the detergent is present at about 0.05-10%, 2-6%, 0.05-0.5%, 0.1-0.15%, 0.1-1.0%, 0.5-1.5%, 1-2%, 1.5-2.5%, 2-3%, 2.5-3.5%, 3-4%, 3.5-4.5%, 4-5%, 4.5-5.5%, 5-6%, 5.5-6.5%, 6-7%, 6.5-7.5%, 7-8%, 7.5-8.5%, 8-9%, or 8.5-9.5% by weight of the composition. In any of the foregoing embodiments, the detergent may comprise a lithium salt. In any of the foregoing embodiments, the detergent may comprise a lauryl sulfate. In any of the foregoing embodiments, the detergent may comprise lithium lauryl sulfate.

### 3. Additives

Compositions of the present disclosure may comprise an additive, e.g., at a concentration from about 0.1 wt % to about 2.0 wt % of the total weight of the composition. Examples of additives include bactericides, biocides, biopolymer degrading agents, bleaching agents, bleaching systems, buffers, chelators, enhancing agents, fungicides, polymers, reagents, salts, preservatives, and stabilizing agents.

### a) Buffers

Buffers may be used to maintain pH of a composition within a desired range. In some embodiments, the desired pH range may be chosen from the following pH ranges: 3 to 11, 3 to 14, 3 to 4, 3.5 to 4.5, 4 to 5, 4.5 to 5.5, 5 to 6, 5.5 to 6.5, 6 to 7, 6.5 to 7.5, 7 to 8, 7.5 to 8.5, 8 to 9, 8.5 to 9.5, 9 to 10, 9.5 to 10.5, 10 to 11, 10.5 to 11.5, 11 to 12, 11.5 to 12.5, 12 to 13, 12.5 to 13.5, and 13 to 14. Examples of buffers include Good's Buffers; alkali salts, ammonia buffer; 3-(N-morpholino)propanesulfonic acid (MOPS); bis(tris(hydroxymethyl)methylamino)propane (BIS-TRIS); borate buffer; carbonate buffer; citrate buffer; hydroxyethyl piperazineethanesulfonic acid (HEPES); phosphate buffer such as sodium phosphate; tartrate buffer; and tris(hydroxymethyl)aminomethane (TRIS) such as TRIS-hydrochloride (TRIS-HCl) and TRIS-EDTA. In some embodiments, the buffer comprises sodium phosphate. In some embodiments, the composition comprises a buffer concentration of 0-0.2% w/v, 0.1-0.3% w/v, 0.2-0.4% w/v, 0.3-0.5% w/v, 0.4-0.6% w/v, 0.5-0.7% w/v, 0.6-0.8% w/v, 0.7-0.9% w/v, 0.8-1.0% w/v, 0.9-1.1% w/v, 1.0-1.2% w/v, 1.1-1.3% w/v, 1.2-1.4% w/v, 1.3-1.5% w/v, 1.4-1.6% w/v, 1.5-0.7% w/v, 1.6-1.8% w/v, 1.7-1.9% w/v, 1.8-2.0% w/v, 1.9-2.1% w/v, 2.0-2.2% w/v, 2.1-2.3% w/v, 2.2-2.4% w/v, 2.3-2.5% w/v, 2.4-2.6% w/v, 2.5-2.7% w/v, 2.6-2.8% w/v, 2.7-2.9% w/v, 2.8-3.0% w/v, 2.9-3.1% w/v, 3.1-3.3% w/v, 3.2-3.4% w/v, 3.3-3.5% w/v, 3.4-3.6% w/v, 3.5-3.7% w/v, 3.6-3.8% w/v, 3.7-3.9% w/v, 3.8-4.0% w/v, 3.9-4.1% w/v, or more. In some embodiments, the buffer concentration is 0.4-0.5% w/v. In some embodiments, the buffer concentration is about 3% w/v.

### b) Chelators

Chelators may be used to inhibit divalent cation-dependent proteases. Examples of chelators include magnesium chelators and calcium chelators. In some embodiments, the chelator is trisodium salt of methyl glycine diacetic acid (MGDA) (Trilon M), trisodium citrate, ethylenediaminetetraacetic acid (EDTA), ethylene glycol-bis(β-aminoethyl ether)-N,N,N',N'-tetraacetic acid (EGTA), or a salt thereof. In some embodiments, the chelator comprises EDTA and EGTA.

In some embodiments, the composition comprises a chelator concentration of at least 20 mg/L, 25 mg/L, 30 mg/L, 35 mg/L, 40 mg/L, 45 mg/L, 50 mg/L, 55 mg/L, 60 mg/L, 65 mg/L, 70 mg/L, 75 mg/L, 80 mg/L, 90 mg/L, 100 mg/L, 200 mg/L, 300 mg/L, 400 mg/L, 500 mg/L or more. In some embodiments, composition comprises a chelator concentration of 200-300 mg/L, 250-350 mg/L, 300-400 mg/L, 350-450 mg/L, 400-500 mg/L, 450-550 mg/L, 500-600 mg/L, 550-650 mg/L, 600-700 mg/L, 650-750 mg/L, 700-800 mg/L, 750-850 mg/L, 800-900 mg/L, 850-950 mg/L, 900-1000 mg/L, or more. In some embodiments, the chelator concentration is 700-800 mg/L.

### c) Salts

Salts may be used to maintain osmolarity of a composition. Exemplary salts used include alkali salt, sodium salt, potassium salt, chloride salt, and acetate salt. In some embodiments, the composition has an osmolarity of 0 to 50 mOsm, 20 to 50 mOsm, 50 to 100 mOsm, 100 to 200 mOsm, 200 to 300 mOsm, 300 to 400 mOsm, 400 to 500 mOsm, 500 to 600 mOsm, 600 to 700 mOsm, 700 to 800 mOsm, or 800 to 1000 mOsm.

### d) Detergent builder, coupling agent, or complexing agent

In some embodiments, the composition may comprise a detergent builder, coupling agent, or complexing agent such as sodium xylene sulfonate, zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates or layered silicates (e.g., SKS-6 from Hoechst), e.g., at a concentration less than or equal to about 65% by weight.

### e) Bleaching system

In some embodiments, the composition may comprise a bleaching system, which may comprise a H₂O₂ source such as perborate, percarbonate, or a peracid-forming bleach activator. In some embodiments, the composition may comprise a peracid-forming bleach activator such as tetraacetylethylenediamine or nonanoyloxybenzenesulfonate. In some embodiments, the bleaching system may comprise peroxyacids of e.g., the amide, imide, or sulfone type.

### f) Stabilizing agents

Stabilizing agents may be used to stabilize enzymes of the composition or target material, such as RNA or DNA. Stabilizing agents may be chosen from boric acid, a boric acid derivative such as an aromatic borate ester or a phenyl boronic acid derivative such as 4-formylphenyl boronic acid, calcium ions, glycerol, lactic acid, magnesium ions, polyethylene glycol, propylene glycol, sodium borate, sugar, sugar alcohol, and suitable enzymes. In some embodiments, the enzyme(s) of the composition may be stabilized, e.g., using a prolylene glycol, polyol such as propylene glycol or glycerol, a sugar or sugar alcohol, lactic acid, boric acid, or a boric acid derivative, e.g., an aromatic borate ester, or a phenyl boronic acid derivative such as 4-formylphenyl boronic acid, and the composition may be formulated as described in e.g., WO 92/19709 and WO 92/19708.

In some embodiments, the stabilizing agent comprises one or more ribonuclease (RNase) inhibitors. RNase inhibitors may inhibit RNase A, RNase B, RNase C, and/or any enzyme that has RNA-hydrolyzing activity. RNase inhibitors include small molecules, enzymes, or antibodies that are used to inhibit RNases from degrading RNA molecules. RNAse inhibitor enzymes and antibodies may be of eukaryotic origin. Exemplary RNase inhibitors include ribonucleosside-vanadyl complex, guanadine thiocyanate, polyvinylsulfonic acid, and human placenta RNase inhibitor. Commercially available RNase inhibitors that may be used include RNeasy, SUPERase·In^{™}, RNaseOUT^{™}, Ambion^{®} RNase Inhibitor, Ambion^{®} RNA*secure*^{™}, RNasin^{®}, RNasin^{®} Plus, RIBOPROTECT, RiboLock, and RiboSafe.

In some embodiments, the stabilizing agent comprises one or more deoxyribonuclease (DNase) inhibitors. DNase inhibitors may inhibit DNase I, DNase II, TREX1 (i.e., DNase III), and/or any enzyme that has DNA-hydrolyzing activity. DNase inhibitors include small molecules, enzymes or antibodies that are used to inhibit DNases from degrading DNA molecules. DNase inhibitors may be of eukaryotic origin or prokaryotic origin. Exemplary DNase inhibitors include DNase inhibitors that originated from human, beef, calf, rabbit, rat, *Nicotiana tabacum,* Streptomyces species, Adenovirus species, *Micromonospora echinospora,* and *Escherichia coli.* Exemplary small molecule DNAse inhibitors include anthracycline and aminoglycoside antibiotics.

### g) Surfactants

In some embodiments, the composition may comprise one or more surfactants which may include non-ionic, anionic, amphoteric, cationic, or a combination of surfactants. In some embodiments, the composition may comprise nonionic surfactants such as alkanolamides, amine oxides, block polymers, ethoxylated primary and secondary alcohols, ethoxylated alkyphenols, ethoxylated fatty esters, sorbitan derivatives, glycerol esters, propoxylated and ethoxylated fatty acids, alcohols, and alkyl phenols, glycol esters, polymeric polysaccharides, sulfates and sulfonates of ethoxylated alkylphenols, and polymeric surfactants. In some embodiments, the composition may comprise anionic surfactants such as ethoxylated amines or amides, sulfosuccinates and derivatives, sulfates of ethoxylated alcohols, sulfates of alcohols, sulfonates and sulfonic acid derivatives, phosphate esters, and polymeric surfactants. In some embodiments, the composition may comprise amphoteric surfactants such as betaine derivatives. In some embodiments, the composition may comprise cationic surfactants such as amine surfactants. In some embodiments, the composition may comprise fluorinated surfactants, such as partially or fully fluorinated alkyl polyethers. In some embodiments, the composition may comprise one or more alcohols, e.g., a c12-c14-secondary ethoxylated alcohol (e.g., Tergitol).

In some embodiments, the concentration of surfactants may vary based on the enzymatic activity of the enzymes, the exposure time of the detergent to the biofilm, whether the detergent is in solid, liquid, spray, or gel form, the medical instrument exposed with the detergent, and the cleaning system utilized. The surfactants may be present in the composition at a concentration of less than about 10% of the total weight of the composition (i.e., wt/wt or wt %). In some embodiments, the surfactant is present at about 0.05-10%, 2-6%, 0.05-0.5%, 0.1-0.15%, 0.1-1.0%, 0.5-1.5%, 1-2%, 1.5-2.5%, 2-3%, 2.5-3.5%, 3-4%, 3.5-4.5%, 4-5%, 4.5-5.5%, 5-6%, 5.5-6.5%, 6-7%, 6.5-7.5%, 7-8%, 7.5-8.5%, 8-9%, or 8.5-9.5% by weight of the composition. In some embodiments, the concentration of surfactants may vary based on the enzymatic activity of the enzymes, the exposure time of the detergent to the biofilm, whether the detergent is in solid, liquid, spray, or gel form, the medical instrument exposed with the detergent, and the cleaning system utilized.

### h) Polymers

In some embodiments, the composition may comprise one or more polymers, such as carboxymethylcellulose, poly(vinylpyrrolidone), poly(ethylene glycol), poly(vinyl alcohol), poly(vinylpyridine-N-oxide), poly(vinylimidazole), and polycarboxylates such as polyacrylates, maleic/acrylic acid copolymers and lauryl methacrylate/acrylic acid copolymers.

### i) Preservatives

In some embodiments, the composition may comprise a preservative. Any compound which may function as a microbicide, bactericide, biocide, or fungicide may be used as a preservative in the composition. Any compound which may maintain the stability of nucleic acids may also be used as a preservative in the composition. In some embodiments, the preservative is benzisothiazolinone (1,2-benzisothiazol-3(2H)-one; BIT).

### j) Other ingredients

In some embodiments, the composition may also contain other ingredients such as clays, foam boosters, suds suppressors, anti-corrosion agents, soil-suspending agents, anti-soil re-deposition agents, dyes, bactericides, hydrotropes, and tarnish inhibitors. In some embodiments, the hydrotropes may be chosen from urea, tosylate, cumenesulfonate, and xylene sulfonate.

### D. Biological Samples

A biological sample may comprise a body fluid obtained from a subject that may contain a target material. Examples of biological samples and target materials are provided in Section B above.

In some embodiments, the biological sample is sputum, saliva, mucoidal secretions, nasal mucus, nasal discharge, pulmonary secretions, a nasopharyngeal swab sample, an oropharyngeal swab sample, bronco alveolar lavage, bronchial washings, urine, blood, plasma, or serum. In some embodiments, any type of pulmonary sample is a suitable biological sample. In some embodiments, the target material comprises nucleic acids and/or respiratory pathogens.

### 1. Mucoidal Secretions

In some embodiments, a biological sample is a type of mucoidal secretion or mucus, which is an aqueous, viscous liquid comprising mucins (i.e., glycol proteins with great water-holding capacity). Mucoidal secretions are produced by mucous cells, which may be organized into mucous glands and/or mucous membranes. Many types of mucoidal secretions may be analyzed for target nucleic acids and/or respiratory pathogens. Examples of mucoidal secretions include nasal secretions, pulmonary secretions, gastric mucus, and vaginal secretions.

### 2. Pharmaceutical Molecules

In some embodiments, a biological sample may contain one or more types of pharmaceutical molecules. For example, a biological sample may be collected from a subject who has been treated with a pharmaceutical molecule. In these instances, the biological sample may be tested to ensure that the pharmaceutical molecule(s) do not interfere with sample storage, processing, or analysis.

In some embodiments, the pharmaceutical molecule is a large molecule, such as an antibody, antibody fragment, antibody conjugate, or any protein that may have a therapeutic effect on the subject. In some embodiments, the pharmaceutical molecule is a small molecule capable of modulating biochemical processes in the subject. In some embodiments, the pharmaceutical molecule is one that may be prescribed for a respiratory infection. In some embodiments, the pharmaceutical molecule is an antibiotic, anti-viral drug, or anti-fungal drug. In some embodiments, the pharmaceutical molecule is one that may alleviate symptoms of an infection, such as a respiratory infection.

### E. Respiratory Pathogens

In some embodiments, a biological sample may comprise target material derived from a respiratory pathogen. Many types of viruses, fungi, and bacteria can infect the respiratory tract. While respiratory infections may share similar symptoms, such as bronchiolitis, chest pain, chills, coughing, croup, fatigue, fever, headache, hypoxia, loss of appetite, muscle or body aches, pneumonia, sore throat, stuffy or runny nose, trouble breathing, and/or whooping cough, treatment can be different depending on the causative agent. It can, therefore, be useful to determine the respiratory pathogen behind the infection.

### 1. Viruses

In some embodiments, the respiratory pathogen is a virus, e.g., an influenza virus. Influenza viruses can cause seasonal epidemic of disease known as the flu season, as well as flu pandemics. Examples of influenza virus include influenza A, influenza B, influenza C, and influenza D.

In some embodiments, the respiratory pathogen is a coronavirus. Coronaviruses can cause respiratory infections and have been responsible for regional and global pandemics. Coronaviruses are classified into four subtypes - alpha, beta, gamma, and delta. Examples of coronaviruses include 229E, HKU1, Middle East Respiratory Syndrome Coronavirus (MERS-CoV), NL63, OC43, Severe Acute Respiratory Syndrome Coronavirus (SARS-CoV), and Severe Acute Respiratory Syndrome Coronavirus 2 (SARS-CoV-2).

In some embodiments, the respiratory pathogen is a paramyxovirus. Examples of paramyxoviruses include respiratory syncytial virus (RSV), including RSV include RSV subtypes A and B; human parainfluenza viruses (HPIV), including HPIV subtypes 1 to 4; and human metapneumovirus (HMPV).

In some embodiments, the respiratory pathogen is an adenovirus. In addition to infections that relate to the respiratory tract, adenoviruses can also cause diarrhea and conjunctivitis. Examples of adenovirus include mammalian adenoviruses (mastadenoviruses) and avian adenoviruses (aviadenoviruses). Examples of adenoviruses that infect humans include adenovirus type A (serotypes 12, 18, and 31), B (serotypes 3, 7, 11, 14, 16, 21, 34, and 35), C (serotypes 1, 2, 5, and 6), D (serotypes 8, 9, 10, 13, 15, 17, 19, 20, 22-30, 32, 33, 36-36, and 42-47), E (serotype 4), and F (serotypes 40 and 41).

In some embodiments, the respiratory pathogen is a rhinovirus. Examples of rhinoviruses include Rhinoviruses A, B, and C.

In some embodiments, the respiratory pathogen is an enterovirus. Examples of enteroviruses include EV-D68 and EV-71.

### 2. Bacteria

In some embodiments, the respiratory pathogen is a Bordetella species, such as *Bordetella pertussis*, which causes whooping cough or pertussis, or *Bordetella para pertussis.*

In some embodiments, the respiratory pathogen is *Staphylococcus aureus.* In some embodiments, the *S. aureus* is methicillin-resistant *S. aureus* (MRSA).

In some embodiments, the respiratory pathogen is *Streptococcus pneumoniae. Streptococcus pneumoniae* infections can range from respiratory tract, ear, and sinus infections to pneumonia and bloodstream infections.

In some embodiments, the respiratory pathogen is a mycobacterium. This genus includes pathogens known to cause serious disease in mammals, including tuberculosis and leprosy in humans. Examples of mycobacterium include *Mycobacterium tuberculosis.*

In some embodiments, the respiratory pathogen is a Chlamydia that is transmitted via small respiratory droplets. In some embodiments, the Chlamydia is *Chlamydia pneumoniae.*

In some embodiments, the respiratory pathogen is *Mycoplasma pneumoniae. Mycoplasma pneumoniae* can cause mild infections of the respiratory system as well as more serious lung infections that require hospitalization.

### 3. Fungi

In some embodiments, the respiratory pathogen is an Aspergillus species, such as *Aspergillus fumigatus.* Infection by Aspergillus species may cause aspergillosis.

In some embodiments, the respiratory pathogen is a Blastomyces species, such as *Blastomyces dermatitidis.* Infection by *Blastomyces dermatitidis* may cause blastomycosis.

In some embodiments, the respiratory pathogen is a Coccidioides species, such as *Coccidioides immitis.* Infection by *Coccidioides immitis* may cause coccidioidomycosis, also called valley fever.

In some embodiments, the respiratory pathogen is a Cryptococcus species, such as *Cryptococcus neoformans.* Infection by *Cryptococcus neoformans* may cause Cryptococcosis.

In some embodiments, the respiratory pathogen is a Histoplasma species, such as *Histoplasma capsulatum.* Infection by *Histoplasma capsulatum* may cause histoplasmosis.

In some embodiments, the respiratory pathogen is a Rhizopus species or Mucor species. Infection by Rhizopus species or Mucor species may cause mucormycosis.

In some embodiments, the respiratory pathogen is a Pneumocystis species, such as *Pneumocystis jirovecii.* Infection by *Pneumocystis jirovecii* may cause Pneumocystis pneumonia.

### F. Gastrointestinal Pathogens

In some embodiments, a biological sample may comprise target material derived from a gastrointestinal pathogen. Many types of viruses, parasites, and bacteria can cause gastrointestinal infections. While gastrointestinal infections may share similar symptoms, such as nausea, vomiting, diarrhea, abdominal pain, fatigue, fever, headache, loss of appetite, and/or muscle or body aches, treatment can be different depending on the causative agent. It can, therefore, be useful to determine the gastrointestinal pathogen behind the infection.

### 1. Bacteria

In some embodiments, the gastrointestinal pathogen is a Yersinia species, such as *Yersinia enterocolitica.* Infection by Yersinia enterocolitica may cause yersiniosis.

In some embodiments, the gastrointestinal pathogen is a Vibrio species, such as *Vibrio parahaemolyticus, Vibrio vulnificus,* and *Vibrio cholerae.* Infection by Vibrio species may cause vibriosis.

In some embodiments, the gastrointestinal pathogen is an Escherichia species, such as *Escherichia coli* 0157, *Escherichia coli* Stx1, and *Escherichia coli* Stx2.

In some embodiments, the gastrointestinal pathogen is a Plesiomonas species, such as *Plesiomonas shigelloides.*

In some embodiments, the gastrointestinal pathogen is a Campylobacter species, such as *Campylobacter coli, Campylobacter lari*, and *Campylobacter upsaliensis.* Infection by Campylobacter species may cause campylobacteriosis.

In some embodiments, the gastrointestinal pathogen is a Shigella species such as *Shigella boydii*, *Shigella dysenteriae*, *Shigella flexneri*, and *Shigella sonnei.* Infection by Shigella species may cause shigellosis.

### 2. Viruses

In some embodiments, the gastrointestinal pathogen is a virus such as norovirus, rotavirus, astrovirus, adenovirus, and sapovirus.

### 3. Parasites

In some embodiments, a biological sample may comprise target material derived from a parasite. While parasitic infections may share similar symptoms, treatment can be different depending on the causative agent. It can, therefore, be useful to determine the parasite behind the infection.

In some embodiments, the parasite is a Giardia species, such as *Giardia intestinalis.* Infection by Giardia species may cause giardiasis.

In some embodiments, the parasite is a Cryptosporidium species, such as *Cryptosporidium parvum.* Infection by Cryptosporidium species may cause cryptosporidiosis.

In some embodiments, the parasite is an Entamoeba species, such as Entamoeba histolytica. Infection by *Entamoeba hystolytica* may cause amebiasis.

In some embodiments, the parasite is a Cyclospora species, such as *Cyclospora cayetanensis.* Infection by *Cyclospora cayetanensis* may cause cyclosporiasis.

In some embodiments, the bloodborne pathogen is Babesia species such as Babesia microti. Infection by Babesia species may cause babesiosis, which is spread by the bite of infected Ixodes scapularis ticks.

### G. Bloodborne Pathogens

In some embodiments, a biological sample may comprise target material derived from a bloodborne pathogen. Bloodborne pathogens may be transmitted when any body fluid, such as blood, saliva, or mucosal secretion, from an infected person enters another person's body via needle-sticks, human bites, cuts, abrasions, or through mucus membranes. Many types of viruses, parasites, and bacteria can cause blood infections.

### 1. Parasites

In some embodiments, the bloodborne pathogen is a Plasmodium, such as *Plasmodium falciparum, Plasmodium malariae*, *Plasmodium ovale,* and *Plasmodium knowlesi.* Infection by Plasmodium may cause malaria.

### 2. Viruses

In some embodiments, the bloodborne pathogen is a hepatitis virus, such as hepatitis B virus (HBV) and hepatitis C virus (HCV). In some embodiments, the bloodborne pathogen is the human immunodeficiency virus (HIV).

### H. Exemplary Methods

### 1. Contacting a biological sample with a swab, wherein an aliquot of the biological sample and the swab become associated; swabs; samples

In some embodiments, methods disclosed herein use a swab associated with an aliquot of a biological sample. In some embodiments, methods according to the disclosure can comprise contacting the biological sample with the swab, wherein an aliquot of the biological sample and the swab become associated. The association between the sample aliquot and the swab should be temporary or reversible at least in part, so that at least some analyte (e.g., cells or nucleic acid) can be released into a liquid. One skilled in the art can choose an appropriate manner of contacting the sample with the swab so as to achieve a suitable association of the sample aliquot with the swab, e.g., dipping, wiping, poking, or spinning the swab into, on, or across the sample.

Any swab suitable for adsorbing and releasing biological material can be used. In some embodiments, a swab is used that comprises a stem with absorbent and/or adsorbent material (e.g., sponge or fibers, which can be, e.g., cotton, alginate, silk, carbon fiber, or polymeric fibers, such as rayon, polyester, or polyamide) wrapped around, bonded, or adhered to an end to form a tip, the absorbent or adsorbent material being suitable for adsorbing and/or absorbing the sample to be collected. In some embodiments, the tip has a thickness (measured perpendicularly to the stem axis) of 0.5 to 3 mm. In some embodiments, the tip comprises fibers having a linear density of 1 to 4 mg per 10 meters. In some embodiments, the swab is a nasal swab.

In some embodiments, a flocked swab is used comprising an elongate support body and a plurality of flocked fibers at an end of the support. Exemplary flocked swabs are described in US 2006/0142668. Swabs can have elongate stems generally made of plastic materials, for example polystyrene.

Any biological sample that can become associated with a swab can be used in methods according to the disclosure. Biological samples used in methods according to the disclosure can be emulsions, suspensions, or solutions (or a combination thereof) and can comprise various types of impurities or contaminants, such as viscous polymers or particulate matter. Viscous polymers can include polysaccharides (e.g., mucopolysaccharides, lipopolysaccharides, proteoglycans, starch, glycogen, or soluble cellulose), extracellular nucleic acids, and polypeptides (including glycoproteins and lipopolypeptides). Particulate matter can include material derived from food, solid lipids, inorganic solids such as phosphates (e.g., calcium or iron phosphate), and insoluble fibrous matter (e.g., cellulose fibers). A sample can comprise a lipid phase or a lipid emulsion. A sample can comprise cellular debris, which depending on its size, solubility, and hydrophobicity may occur at least in part as particulate matter, viscous polymers, a lipid phase or emulsion, or a combination thereof. Samples can be crude or may have undergone some initial purification, such as filtration, extraction, or fractionation (e.g., chromatographic). In some embodiments, a crude sample is diluted, suspended, or at least partially dissolved with a suitable liquid such as water or a solution, which may be buffered, saline, or isotonic with the sample.

Exemplary sample types that may include particulate matter, viscous polymers, or emulsions are sputum, saliva, mucoidal secretions, mucus such as nasal mucus, pulmonary secretions, blood, plasma, serum, urine, soil, sludge, stool, or a microbial growth. Sludge and soil include natural and artificial versions thereof, such as mud, potting mix, chemical waste, etc. Microbial growths include bacterial, archaeal, protist, or fungal growths, such as colonies, biofilms, mycelia, etc. In some embodiments, the sample is a food sample.

In some embodiments, a sample comprises or is suspected of comprising a pathogen, virus, fungus, or bacterium. In some embodiments, the pathogen is a respiratory tract pathogen. Examples of respiratory pathogens are discussed in Section E above.

Any volume of material sufficient to give an adequate second portion for the intended downstream use, as the case may be, can be associated with the swab. In some embodiments, a volume of 20 to 550 microliters of sample, such as 20 to 50, 50 to 100, 100 to 150, 150 to 200, 200 to 250, 250 to 300, 300 to 350, 350 to 400, 450 to 500, or 500 to 550 microliters, or 20 to 75, 75 to 125, 125 to 175, 175 to 225, 225 to 275, 275 to 325, 325 to 375, 375 to 425, 425 to 475, 475 to 525, or 525 to 575 microliters becomes associated with the swab.

### 2. A composition for stabilizing nucleic acids

In some embodiments, the composition in the methods disclosed herein are useful for stabilizing nucleic acids in a biological sample such that the nucleic acids may be preserved or protected from degradation. In some embodiments, the composition stabilizes DNA. In some embodiments, the composition stabilizes RNA. The composition may be used according to the methods disclosed herein at any temperature. In some embodiments, the composition is mixed with the biological sample at room temperature.

In some embodiments, the composition is used as a diluent when it is mixed with a biological sample. While, the composition may be mixed with any biological sample, in some embodiments, the biological sample is whole blood or urine.

In some embodiments, the composition is used as a matrix when it is mixed with a biological sample. For example, the composition may be used to prepare RNA transcripts for testing in RNA panels.

In some embodiments, the composition is used as storage media for a biological sample or for the nucleic acids associated with a biological sample.

### 3. A composition for inactivating infectious pathogens

In some embodiments, the composition in the methods disclosed herein are useful for inactivating infectious pathogens in a biological sample such that the pathogens are less capable or no longer capable of causing disease. In some embodiments, the composition disrupts the pathogen's ability to replicate. In some embodiments, the composition disrupts the pathogen's ability to infect a host. In some embodiments, the composition may cause lysis of the pathogen.

### 4. Further processing and/or analysis of sample material including cells, nucleic acids, or polypeptides; capture, amplification, and detection of targets; target bacterial or pathogen sequences

In some embodiments, viruses or cells (e.g., bacteria) released into the liquid are disrupted or lysed, e.g., by heating, sonication, vortexing, osmolysis, proteolysis, exposure to alkali and/or detergent, or a combination thereof. This can occur in the liquid, either commencing immediately upon transferring the swab into the liquid such as in the case of the liquid being a lysis solution, or afterward, e.g., as in the case of further manipulation by addition of reagents or use of equipment following release of the portion of the aliquot into the liquid. In some embodiments, nucleic acids are isolated from the lysate, e.g., by chromatography or precipitation, or binding to a binding agent, such as at least one capture oligomer.

In some embodiments, nucleic acids released from the swab into the carrier liquid or from cells or viruses released and lysed as discussed above are further isolated or purified. For example, further isolation or purification can be performed using chromatography or precipitation, or by binding nucleic acid to a binding agent, such as at least one capture oligomer. For example, it can be beneficial to further isolate or purify nucleic acids intended to be subjected to downstream analytical approaches, e.g., amplification or sequencing, that may otherwise be inhibited in whole or part due to impurities.

Where at least one capture oligomer is used, it can be sequence-specific (i.e., it comprises a target-hybridizing region configured to specifically hybridize to a site in the target nucleic acid to form a duplex). One example of a capture oligomer includes two binding regions: a sequence-binding region (e.g., target-specific portion) and an immobilized probe-binding region, usually on the same oligomer, although the two regions may be present on two different oligomers joined together by one or more linkers. Alternatively, capture oligomers with randomized, repeating, or non-specific sequences for hybridization to a target can be used, such as poly-(k) and poly-(r) capture oligomers and combinations thereof, which are described, e.g., in US 2013/0209992 and in US 2020/0165599. In some embodiments, a capture oligomer uses a target-sequence binding region that includes random or non-random poly-GU, poly-GT, or poly-U sequences to bind non-specifically to a target nucleic acid. In some embodiments, a capture oligomer uses a target-sequence binding region that includes random or non-random poly-GA, sequences to bind non-specifically to a target nucleic acid. In some embodiments, a combination of capture oligomers is used in which a first capture oligomer comprises random or non-random poly-GA, sequences and the second capture oligomer comprises random or non-random poly-GU or poly-GT sequences. The capture oligomers can further comprise a sequence or moiety that binds an immobilized probe on a support.

In some embodiments, a method further comprises determining the presence or absence of at least one macromolecule in the sample. In some embodiments, nucleic acids isolated from the lysate can be subjected to one or more analytical procedures such as detection of the presence or absence of one or more target sequences (e.g., using one or more probes, such as probe oligomers, for the target regions, which can follow amplification of the target sequences or can be performed on isolated nucleic acid directly, as in Southern, slot, or dot blotting); sequencing; electrophoresis; molecular cloning; or microarray analysis. In some embodiments, an amplification reaction is performed using isothermal amplification or amplification methods that involve temperature cycling, such as polymerase chain reaction (PCR) and transcription-mediated amplification (TMA), or any other type of amplification including those discussed above.

In some embodiments, a probe oligomer or FRET cassette is used that comprises a label. Particularly suitable labels include compounds that emit a detectable light signal, e.g., fluorophores or luminescent (e.g., chemiluminescent) compounds that can be detected in a homogeneous mixture. More than one label, and more than one type of label, may be present on a particular probe, or detection may rely on using a mixture of probes in which each probe is labeled with a compound that produces a detectable signal (see. e.g., U.S. Pat. Nos. 6,180,340 and 6,350,579). Labels may be attached to a probe by various means including covalent linkages, chelation, and ionic interactions, but in some embodiments the label is covalently attached. For example, in some embodiments, a detection probe has an attached fluorescent or chemiluminescent label such as, e.g., an acridinium ester (AE) compound (see. e.g., U.S. Pat. Nos. 5,185,439; 5,639,604; 5,585,481; and 5,656,744), which in typical variations is attached to the probe by a non-nucleotide linker (see. e.g., U.S. Pat. Nos. 5,585,481; 5,656,744; and 5,639,604). In some embodiments, a probe oligomer is labeled with an interactive pair of detectable labels. Examples of detectable labels that are members of an interactive pair of labels include those that interact with each other by FRET or non-FRET energy transfer mechanisms. Fluorescence resonance energy transfer (FRET) involves the radiationless transmission of energy quanta from the site of absorption to the site of its utilization in the molecule, or system of molecules, by resonance interaction between chromophores, over distances considerably greater than interatomic distances, without conversion to thermal energy, and without the donor and acceptor coming into kinetic collision. The "donor" is the moiety that initially absorbs the energy, and the "acceptor" is the moiety to which the energy is subsequently transferred. In addition to FRET, there are at least three other "non-FRET" energy transfer processes by which excitation energy can be transferred from a donor to an acceptor molecule. In some embodiments, the probe oligomer is a probe primer and may be used for nucleic acid amplification.

In some embodiments, at least one probe system is used, wherein the oligomers of the system function together to facilitate detection of a target sequence. For example, in INVADER or INVADER PLUS assays, an invasive oligomer (which can also but does not necessarily function as an amplification oligomer, e.g., primer), a primary probe, and a FRET cassette can be used. INVADER and INVADER PLUS assays are discussed in detail, e.g., in US 2018/0163259.

A detection probe oligomer in accordance with the present disclosure can comprise a non-target-hybridizing sequence. In some applications, probes exhibiting at least some degree of self-complementarity are desirable to facilitate detection of probe:amplicon duplexes in a test sample without first requiring the removal of unhybridized probe prior to detection. Specific embodiments of such detection probes include, for example, probes that form conformations held by intramolecular hybridization, such as conformations generally referred to as hairpins. Particularly suitable hairpin probes include a "molecular torch" (see. e.g., U.S. Pat. Nos. 6,849,412; 6,835,542; 6,534,274; and 6,361,945) and a "molecular beacon" (see. e.g., U.S. Pat. Nos. 5,118,801, 5,312,728, and 5,925,517). In yet other embodiments, a detection probe is a linear oligomer that does not substantially form conformations held by intramolecular bonds.

In some embodiments, an amplification and detection procedure is performed such as an INVADER or INVADER PLUS assay, a TaqMan assay, or a TMA reaction with probe hybridization and detection (e.g., using at least one probe oligomer or FRET cassette comprising a fluorophore and a FRET partner and exhibiting hybridization-dependent and/or degradation-dependent fluorescence). In some embodiments, detection is in real time during the amplification reaction. In some embodiments, detection occurs near, at, or after the end of the amplification reaction. In some embodiments, at least one target sequence is quantified, e.g., based on a standard curve and/or one or more calibration standards.

Embodiments of fluorophores include those that absorb light in the range of about 495 to 650 nm and emit light in the range of about 520 to 670 nm, which include those known as FAM^{™}, TET^{™}, CAL FLUOR^{™} (Orange or Red), and QUASAR^{™} compounds. Fluorophores may be used in combination with a quencher molecule that absorbs light when in close proximity to the fluorophore to diminish background fluorescence. Such quenchers are well known in the art and include, e.g., BLACK HOLE QUENCHER^{™} (or BHQ^{™}) or TAMRA^{™} compounds.

Examples of interacting donor/acceptor label pairs that may be used in connection with the disclosure, making no attempt to distinguish FRET from non-FRET pairs, include fluorescein/tetramethylrhodamine, IAEDANS/ fluororescein, EDANS/DABCYL, coumarin/DABCYL, fluorescein/ fluorescein, BODIPY FL/BODIPY FL, fluorescein/DABCYL, lucifer yellow/DABCYL, BODIPY/DABCYL, eosine/DABCYL, erythrosine/DABCYL, tetramethylrhodamine/DABCYL, Texas Red/DABCYL, CY5/BH1, CY5/BH2, CY3/BH1, CY3/BH2 and fluorescein/QSY7 dye. Those having an ordinary level of skill in the art will understand that when donor and acceptor dyes are different, energy transfer can be detected by the appearance of sensitized fluorescence of the acceptor or by quenching of donor fluorescence. Non-fluorescent acceptors such as DABCYL and the QSY7 dyes advantageously eliminate the potential problem of background fluorescence resulting from direct (i.e., non-sensitized) acceptor excitation. Exemplary fluorophore moieties that can be used as one member of a donor-acceptor pair include fluorescein, ROX, and the CY dyes (such as CY5). Exemplary quencher moieties that can be used as another member of a donor-acceptor pair include DABCYL and the BLACK HOLE QUENCHER moieties which are available from Biosearch Technologies, Inc., (Novato, Calif.).

In some embodiments, an amplification and detection procedure, e.g., quantitative PCR (qPCR), reverse transcriptase PCR (RT-PCR), or nucleic acid sequencing, is performed using a non-specific DNA-binding dye. Examples of non-sequence specific DNA-binding dyes include SYBR^{®} Green I, EvaGreen, SYTO 11, SYTO 13, SYTO 16, SYTO 17, SYTO 21, SYTO 24, SYTO 59, SYTO 60, SYTO 61, SYTO 62, SYTO 63, SYTO 64, SYTO 80, SYTO 81, SYTO 82, and SYTO 83. Examples of non-sequence specific DNA-binding dyes used in sequencing reactions include dye-labeled terminators such as d-rhodamine ddNTPs, BigDye^{®} ddNTPs, and MagaDye^{™} ddNTPs. Examples of non-sequence specific dyes used in microarrays include infrared (IR) dyes, near infrared (NIR) dyes, rhodamine, cyanines (e.g., Cy3, Cy5, and Cy7), fluorescein isothiocyanates (e.g., FITC, Alexa 532, JOE, Oregon Green), BODIPY dyes (e.g., BODIPY 576/589 and BODIPY 630/650).

Where the sample comprises or is suspected of comprising a pathogen, the method can comprise determining the presence or absence of at least one nucleic acid characteristic of the pathogen.

In some embodiments, polypeptides isolated from the lysate can be subjected to one or more analytical procedures such as detection of the presence or absence of one or more target or other forms of characterization, which may include one or more of an antibody or aptamer-binding assay (e.g., ELISA or Western blot); electrophoresis; amino acid sequencing; or mass spectrometry. In some embodiments, at least one polypeptide is quantified, e.g., based on a standard curve and/or one or more calibration standards.

In some embodiments, cells released into the carrier liquid are fixed, e.g., with an aldehyde or other cross-linking agent. In some embodiments, the cells are stained, e.g., with a fluorescent dye or chromophore, which is optionally linked to a binding agent such as a nucleic acid probe (e.g., FISH probe), antibody, or aptamer. In some embodiments, the cells are subjected to flow cytometry, microscopy, or other optical analysis.

### 5. Cell culture and testing of pathogen inactivation

In some embodiments, a formulation is tested for its ability to inactivate one or more pathogens. In some embodiments, the pathogen is a bacterial pathogen, such as those disclosed herein, including *Staphylococcus aureus* and *Bordetella pertussis.* In some embodiments, the bacterial pathogen is assessed for colony formation on an agar plate. In some embodiments, the bacterial pathogen is assessed for hemolytic activity. After the bacterial pathogen is processed with a formulation of interest, the bacterial pathogen is applied to a blood agar plate and assessed for lysis of red blood cells. In some embodiments, the bacterial pathogen is applied at different concentrations or in a series of dilutions. The blood agar plates may be incubated (1) at a range of temperatures, including 20°C, 24°C, 30°C, and 37°C, and (2) for a period of time, including 15 minutes, 1 hour, 2 hours, 4 hours, 24 hours, overnight, or longer.

In some embodiments, the pathogen is a viral pathogen, such as those disclosed therein, including coronavirus 229E and adenovirus serotype 2. In some embodiments, the viral pathogen is assessed for plaque formation. After the viral pathogen is processed with a formulation of interest, the viral pathogen is applied to a cell or tissue type and cytopathic effects (CPE), e.g., the formation of virus plaques, are assessed. In some embodiments, the viral pathogen is applied at different concentrations or in a series of dilutions.

In embodiments where a cell or tissue type is used, the cell or tissue type is grown to about 80-90% confluence before the viral pathogen is applied to the cell or tissue type. Any suitable cell or tissue type may be used, and each cell or tissue type is cultured according to materials and methods known to one of ordinary skill in the art. In some embodiments, the cell or tissue type is of mammalian origin or human origin. In some embodiments, the cell or tissue type is MRC-5.

### I. Kits

Also disclosed is a kit containing any one of the compositions described herein. In some embodiments, the composition is contained within a tube, e.g., a tube comprising a cap.

In some embodiments, the kit further comprises a swab comprising a tip portion and a stem portion. In some embodiments, the tip portion of the swab is a flocked tip. In some embodiments, the stem portion of the swab is a flexible plastic stem. In some embodiments, the swab is a nasopharyngeal swab.

In some embodiments, the kit contains from 1 mL to 10 mL of any one of the compositions described herein. In some embodiments, the composition comprises from 2%-4% (w/v) of propylene glycol, from 2% to 10% (w/v) of a degrative enzyme that is a protease, and about 1% to 4% of an alkyl sulfate detergent, preferably a dodecyl sulfate detergent. In some embodiments, the composition comprises 2%-3% (w/v) of a Good's Buffer and from 0.05%-0.3% (w/v) of a surfactant. A Good's buffer refers to any of the series of 20 dipolar (zwitterionic) buffers developed by Dr. Norman Good (see Good et al., Biochemistry 5(2):467-477 (1966) such as HEPES, BES, bicine, CAPS, CHES, MES, MOPS, Tricine, etc., and also to the 6 subsequently developed zwitterionic buffers AMPSO, CAPSO, HEPPSO, MOPSO, and POPSO. In some embodiments, the Good's Buffer is HEPES.

In some embodiments, the kit further comprises instructions for use. In some embodiments, the instructions for use are provided as a digital document. In some embodiments, the instructions for use include directions for biological sample collection. In some embodiments, the instructions for use include directions for nasopharyngeal sample collection.

Also provided herein is the use of any one of the kits described herein for collection of a biological sample for diagnostic testing. In some embodiments, the biological sample is collected for nucleic acid diagnostic testing. In some embodiments, the biological sample comprises one or more of protein, mucin, sputum, saliva, nasal discharge, mucoidal secretions, nasal mucus, pulmonary secretions, urine, blood, plasma, or serum. In some embodiments, the biological specimen is collected for diagnostic testing to determine the presence or absence of a bacterial pathogen. In some embodiments, the biological sample is collected for diagnostic testing to determine the presence or absence of a respiratory pathogen. In some embodiments, the respiratory pathogen is one or more of a coronavirus, parainfluenza virus (HPIV), Influenza virus, adenovirus, human metapneumovirus (HMPV), rhinovirus, enterovirus, or Respiratory Syncytial Virus.

### EXAMPLES

### Example 1: Effect of Enzyme Containing Detergent and Sample Transport Media on Sample Viscosity and the Amplification and Detection of Target Nucleic Acids

This example compares the effect of treating samples with the following three conditions on decreasing sample viscosity (i.e., breaking up sample clots) and preserving RNA stability for subsequent amplification and detection: (1) commercially available Endozime XP (Ruhof Corporation, cat. no. 34530) diluted to 50%, (2) commercially available Endozime AW Triple Plus with APA (unscented) (Endo; Ruhof Corporation, cat. no. 34521), and (3) sample transport media (STM). The recipe for STM is as follows: 2.07 g/L sodium phosphate, monobasic, monohydrate; 30 g/L lithium lauryl sulfate (LLS); 0.372 g/L EDTA, disodium, dihydrate; 0.38 g/L EGTA, free acid; and 2.13 g/L sodium phosphate, dibasic.

100 mL of each specimen condition was spiked with FluA/B/RSV *in vitro* transcript (IVT) as shown in Table 1.

| **Table 1: IVT Spike per 100 mL of Specimen Condition.** | | | | | |
|---|---|---|---|---|---|
| Desired copies of transcript per 360 µL | Stock solution: copies per µL | Desired copies per µL | Total volume (µL) | Volume of stock IVT (µL) | Volume of specimen condition (µL) |
| 2,000 | 10,000 | 5.56 | 100,000 | 55.56 | 99,944 |

Clinical specimens from thirty healthy volunteers were collected directly into the specimen conditions using nasal swabs (i.e., collected samples in Table 4). Thirty clinical specimens were collected for each specimen condition. Spun samples were loaded on a Panther Fusion instrument for nucleic acid extraction, amplification, and detection. Target nucleic acid extraction, amplification, and detection was performed using the Panther Fusion Flu A/B/RSV assay generally according to manufacturer's instructions (Hologic, Inc., cat. no. PRD-04328).

Aspiration and dispense pipetting performance was monitored for each sample during transfer to the Panther Fusion extraction assembly. Samples that caused a pipetting failure were detected by real-time pressure monitoring and further processing of those samples was aborted. Data was as shown in Table 2, wherein "invalid rate" indicates the fraction of samples that caused a pipetting failure.

| **Table 2: Aspiration/Dispense Data.** | | | |
|---|---|---|---|
| **Formulation** | **n** | **Aspiration/Dispense Result** | |
| 50% Endozime XP (AC in Tables 3 and 4) in water | 30 | 0 clots | 0% invalid rate |
| 100% Endozime AW Triple Plus with APA (unscented) (Endo in Tables 3 and 4) | 30 | 0 clots | 0% invalid rate |
| STM | 30 | 9 clots | 30% invalid rate |

Samples in 100% Endozime AW Triple Plus with APA (unscented) and 50% Endozime XP in water did not have high viscosity and/or clots and were therefore transferred to the Panther Fusion extraction assembly without obstruction.

Nine samples in STM had high viscosity and/or clots which obstructed aspiration and dispensing as indicated by a real-time pressure monitoring. These samples were processed for loading onto the assembly again. In 1 sample, the obstruction was resolved on the second loading attempt. In 5 other samples, the obstructions were resolved after 15 seconds of human intervention by vortexing, and the samples were successfully loaded onto the assembly on the third attempt. Three samples had unresolved clots despite all of the attempts described above. The results demonstrate that the use of a multi-enzyme detergent can decrease sample viscosity and improve sample loading while the use of STM did not.

Next, amplification and detection reactions were performed on the samples. Reactions were performed at about 12 and 16 hours after the samples were spiked with IVT. As shown in Table 4, amplification and detection of target nucleic acids was poor for samples in 50% Endozime XP or 100% Endozime AW Triple Plus with APA (unscented) (Endo). Of the samples in AC or Endo that tested positive for Flu A, RSV, or Flu B, the Mean Cts did not demonstrate comparable Ct or analyte detection compared to STM when sample matrix was introduced into the media.

In contrast, target nucleic acids were successfully amplified and detected in all samples in STM (Table 3). These results were consistent with results from positive control reactions without clots (Table 4), in which the target nucleic acids for Influenza A (Flu A), Influenza B virus (Flu B), and Respiratory Syncytial Virus A/B (RSV) were detected in all the reactions that were performed at about 0, 12, and 16 hours after they were spiked with IVT.

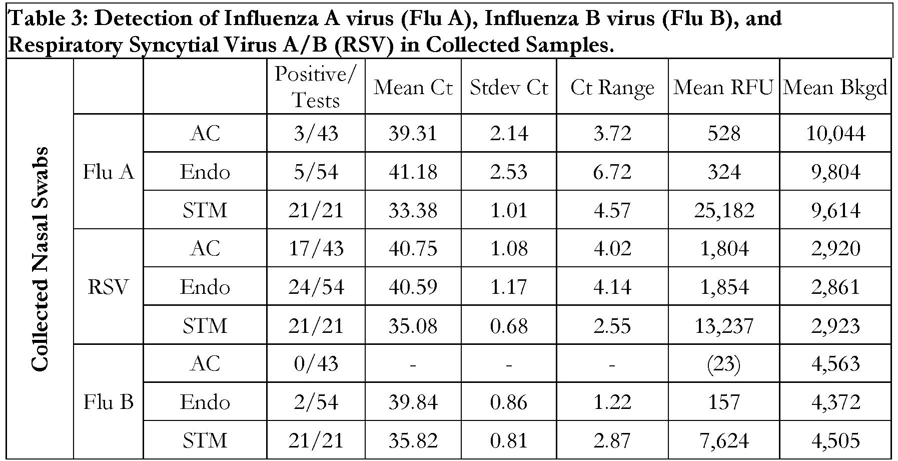

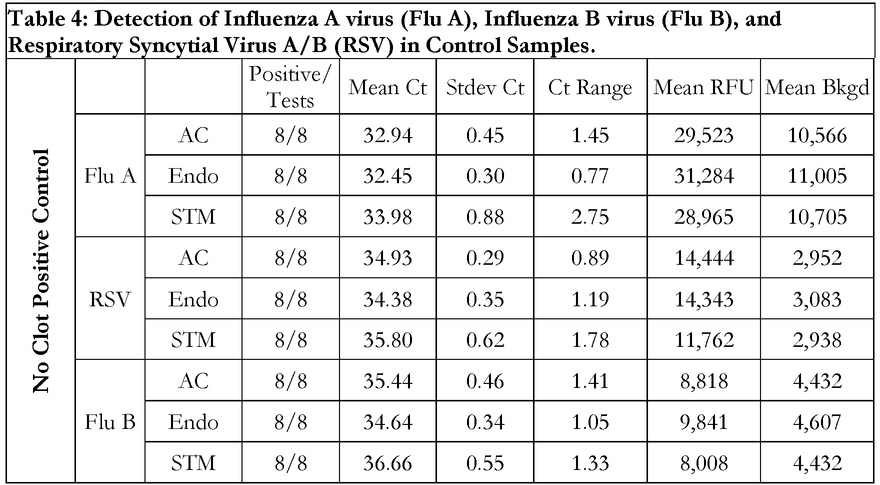

The data show that while the presence of an enzyme containing detergent may decrease sample viscosity to aid sample processing, it did not effectively stabilize target nucleic acids.

### Example 2: Aspiration of Viscous Samples and/or Samples Containing Non-Homogenous Content Following Collection into Several Sample Transport Media

The purpose of this example was to reduce aspiration failure associated with specimen containing mucoid or other viscous or non-homogenous material. Clinical specimens from thirty healthy volunteers were collected using nasal swabs. Following collection, the swabs were each individually placed into 1 ml of STM. The recipe for STM is as described above in Example 1. Further, each collected clinical specimen in a specimen condition was agitated and then centrifuged at 200 RCF for 1 minute. Additionally, each ml of STM contained about 6.9 copies/µL of IVT for each of Influenza A, Influenza B, and respiratory syncytial virus target nucleic acid. The collected specimens were inspected for the presence of blood and ranked as -Blood, +Blood, or ++Blood, depending on whether the specimen contained no blood, trace amounts of blood, or visible blood clots, respectively. The total volume was then brought to 2.9 mL using additional STM or a combination of STM and Endozime AW Triple Plus with APA, a multi-enzymatic detergent. The resulting sample transport media contained 0% Endozime AW Triple Plus with APA (i.e., STM only), 50% Endozime AW Triple Plus with APA, or 65% Endozime AW Triple Plus with APA (Table 5).

| **Table 5: Specimen Conditions.** | | |
|---|---|---|
| | Volume of STM | Volume of Endozime AW Triple Plus with APA |
| STM | 2.9 mL | 0 mL |
| 50% Endozime AW Triple Plus with APA in STM ("50%" in Tables 7-9) | 1.45 mL | 1.45 mL |
| 65% Endozime AW Triple Plus with APA in STM ("65%" in Tables 7-9) | 1.02 mL | 1.88 mL |

Thirty (30) clinical samples were prepared as described in Example 1, and each clinical specimen was divided into each of the three specimen conditions. Real-time pressure monitoring data were collected as described in Example 1.

In thirty specimens collected into STM, there were 6 clots associated with pipetting failures. Thirty specimens collected in 50% Endozime AW Triple Plus with APA resulted in one clot associated with a pipetting failure. Thirty specimens collected into 65% Endozime AW Triple Plus with APA resulted in no clots and aspiration of all thirty specimens was successful. These data show that the addition of a multi-enzymatic detergent to the STM reduced viscosity and non-homogenous content in a sample and reduced the frequency of pipetting failures.

### Example 3: Amplification and Detection of Viscous Samples and/or Samples Containing Non-Homogenous Content Following Collection into Several Sample Transport Media

Clinical specimens from Example 1 were spiked with whole blood. Then, the specimens were amplified and analyzed as described in Example 1 to determine the presence or absence of the spiked IVTs in each specimen condition. Extraction, amplification, and detection was performed using the Panther Fusion Flu A/B/RSV Assay generally as described by the manufacturer.

Results from this example are presented in Tables 6-8. Conditions used and the presence/absence of blood in the sample are indicated. These results show that target nucleic acids were amplifiable and detectable in all three conditions that were tested (as shown in Table 5). These results also show roughly equivalent Counts (Cts) but lower standard deviations for the specimen processed in the 50% Endozime AW Triple Plus with APA (50%) and the 65% Endozime AW Triple Plus with APA (65%) compared to those processed in the STM.

| **Table 6: Detection of the Influenza A virus (Ct).** | | | | | | | |
|---|---|---|---|---|---|---|---|
| | | **N** | **Dropouts** | **Ct Mean** | **Ct Stdev** | **Mean RFU** | **Mean Bkgd** |
| **Panel** | **-Blood** | **12** | **0** | **37.11** | **0.34** | **8,886** | **3,353** |
| STM | -Blood | 9 | 1 | 34.29 | 1.19 | 16,806 | 10,324 |
| | +Blood | 3 | 0 | 33.45 | 0.40 | 28,453 | 12,155 |
| | ++Blood | 3 | 0 | 35.21 | 2.12 | 14,559 | 10,978 |
| 50% | -Blood | 3 | 0 | 34.17 | 0.58 | 23,402 | 11,507 |
| | +Blood | 1 | 0 | 33.35 | - | 23,481 | 12,195 |
| | ++Blood | 1 | 0 | 40.63 | - | 1,547 | 9,285 |
| 65% | -Blood | 5 | 0 | 34.16 | 0.70 | 14,508 | 11,239 |
| | +Blood | 4 | 0 | 33.30 | 0.16 | 23,228 | 11,626 |
| | ++Blood | 1 | 0 | 33.51 | - | 13,698 | 11,979 |

| **Table 7: Detection of the Influenza B virus (Ct).** | | | | | | | |
|---|---|---|---|---|---|---|---|
| | | **N** | **Dropouts** | **Ct Mean** | **Ct Stdev** | **Mean RFU** | **Mean Bkgd** |
| **Panel** | **-Blood** | **12** | **1** | **38.01** | **1.23** | **3,554** | **4,247** |
| STM | -Blood | 9 | 1 | 36.55 | 1.01 | 3,081 | 4,519 |
| | +Blood | 3 | 0 | 36.12 | 0.57 | 4,895 | 4,917 |
| | ++Blood | 3 | 0 | 37.79 | 2.41 | 2,608 | 4,724 |
| 50% | -Blood | 3 | 0 | 37.33 | 2.16 | 3,583 | 4,679 |
| | +Blood | 1 | 0 | 36.45 | - | 3,613 | 5,108 |
| | ++Blood | 1 | 1 | - | - | 55 | 3,904 |
| 65% | -Blood | 5 | 0 | 35.55 | 0.47 | 3,451 | 4,740 |
| | +Blood | 4 | 0 | 35.41 | 0.77 | 5,032 | 4,810 |
| | ++Blood | 1 | 0 | 36.84 | - | 1,738 | 4,983 |

| **Table 8: Detection of the Respiratory Syncytial Virus A/B (Ct).** | | | | | | | |
|---|---|---|---|---|---|---|---|
| | | **N** | **Dropouts** | **Ct Mean** | **Ct Stdev** | **Mean RFU** | **Mean Bkgd** |
| **Panel** | **-Blood** | **12** | **0** | **37.11** | **0.34** | **8,886** | **3,353** |
| STM | -Blood | 9 | 1 | 37.39 | 1.36 | 6,001 | 3,516 |
| | +Blood | 3 | 0 | 36.53 | 0.31 | 8,592 | 3,922 |
| | ++Blood | 3 | 0 | 37.67 | 1.42 | 6,281 | 3,780 |
| 50% | -Blood | 3 | 0 | 37.31 | 0.92 | 6,417 | 3,608 |
| | +Blood | 1 | 0 | 36.38 | - | 8,923 | 3,863 |
| | ++Blood | 1 | 0 | 41.03 | - | 1,923 | 2,875 |
| 65% | -Blood | 5 | 0 | 36.48 | 0.42 | 7,811 | 3,773 |
| | +Blood | 4 | 0 | 36.04 | 0.29 | 8,941 | 3,824 |
| | ++Blood | 1 | 0 | 37.22 | | 5,989 | 3,870 |

### Example 4: Assessment of Assay Performance in the Presence of a Multi-Enzyme Detergent

A further set of assays were performed using the STM, 50% Endozime AW Triple Plus with APA (50%), and 65% Endozime AW Triple Plus with APA (65%) conditions as described above in Example 2 and Table 6 except that patient specimens (nasal swabs from volunteers) was not included in the samples. About 6.9 copies/mL of IVT for each of Influenza A (Flu A), Influenza B (Flu B), and respiratory syncytial virus (RSV) target nucleic acid was spiked into each sample. Target nucleic acid extraction, amplification, and detection was performed on a Panther Fusion system using the Panther Fusion Flu A/B/RSV assay according to manufacturer's instructions. As shown in Table 9, results show equal or lower Cts for target nucleic acids spiked in to the 50% or 65% Endozime AW Triple Plus with APA, compared with those spiked into the 0% Endozime AW Triple Plus with APA (i.e., STM in Table 9), indicating that use of a multi-enzymatic detergent such as Endozime AW Triple Plus with APA in combination with STM did not negatively impact assay performance.

| **Table 9: Detection of Influenza A virus (Flu A), Influenza B virus (Flu B), and Respiratory Syncytial Virus A/B (RSV).** | | | | | | |
|---|---|---|---|---|---|---|
| | | Positive/Tests | Mean Ct | Stdev Ct | Mean RFU | Mean Bkgd |
| Flu A | 50% | 12/12 | 34.06 | 0.45 | 26,985 | 11,115 |
| | 65% | 12/12 | 33.91 | 0.89 | 26,344 | 10,995 |
| | STM | 12/12 | 34.54 | 0.58 | 26,237 | 11,306 |
| | STM (12/8/2020) | 12/12 | 34.57 | 0.49 | 27,285 | 11,448 |
| RSV | 50% | 12/12 | 36.48 | 0.59 | 9,627 | 3,767 |
| | 65% | 12/12 | 36.07 | 0.66 | 9,970 | 3,482 |
| | STM | 12/12 | 37.06 | 0.46 | 8,500 | 3,486 |
| | STM (12/8/2020) | 12/12 | 37.11 | 0.34 | 8,886 | 3,353 |
| Flu B | 50% | 12/12 | 36.35 | 0.54 | 5,536 | 4,827 |
| | 65% | 12/12 | 36.16 | 1.05 | 5,345 | 4,533 |
| | STM | 11/12 | 37.53 | 0.73 | 4,146 | 4,415 |
| | STM (12/8/2020) | 11/12 | 38.01 | 1.23 | 3,554 | 4,247 |

### Example 5: Enhanced STM (E-STM) for Improved Sample Viscosity

Based on formulations and observations discussed in the preceding examples, new formulations were designed and tested for improvements in the following areas: (1) ability to reduce clots in biological samples, (2) stabilizing nucleic acids when specimens are collected and stored in the formulation, and (3) inactivation of pathogens (4) formation of a homogenous solution while stored at various temperatures; and (5) stability of at room temperature for at least 1 year.

This example discusses changes in formulation and how those changes affect sample viscosity. A set of assays were performed as described in Example 1 above using STM, 65% Endozime XP, 65% Endozime BioClean in STM, and 65% Elementum AW. The STM formulation was as described in Example 1 above. Data from the assays was as shown in Table 10 below. The presence of Endozime BioClean and Elementum AW reduced clot errors compared to the STM control, without manipulation of the samples, such as vortexing.

| **Table 10. Aspiration/Dispense Data.** | | | |
|---|---|---|---|
| **Formulation** | **n** | **Aspiration/Dispense Result** | |
| STM | 15 | 6 clots | 5/6 clots resolved with vortex |
| 65% Endozime XP | 15 | 8 clots | All clots were resolved after vortex |
| 65% Endozime BioClean in STM | 15 | 1 clot | Clot was resolved with vortex |
| 65% Elementum AW | 15 | 1 clot | Resolved with vortex |

### Example 6: E-STM Shows Improved Nucleic Acid Preservation

This example discusses formulation changes and how those changes affect nucleic acid stability in the samples. Flu A, RSV, and Flu B IVTs were spiked into one of 100% STM, 100% Endozime BioClean, 100% Endozime XP, 100% Elementum, 65% Endozime BioClean, 65% Endozime XP, or 65% Elementum AW. Formulations with 65% Endozime BioClean, 65% Endozime XP, or 65% Elementum AW were each diluted from 100% to 65% using (1) 2 parts Endozime BioClean, Endozime XP, or Elementum AW, and (2) 1 part STM. Because STM contained 3% (w/v) LLS, the E-STM formulations with 65% Endozime BioClean, 65% Endozime XP, or 65% Elementum AW each contained a final concentration of 1% (w/v) LLS. The stability of nucleic acids in these formulations were compared. The experiments were carried out as described in Example 1 above.

As shown in Table 11 below, the 100% Endozime BioClean, 100% Endozime XP, and 100% Elementum AW formulations did not sufficiently stabilize nucleic acids in the presence of patient swabs. However, as shown in Table 12 below, STM formulations supplemented with 65% Endozime BioClean or 65% Endozime XP did stabilize nucleic acids in the presence of patient swabs. It was noted that the STM formulation supplemented with 65% Endozime BioClean containing 1% (w/v) LLS reduced CLT errors (example 5) and showed stabilization of the nucleic acids in the samples, facilitating amplification and detection.

| **Table 11. Detection of FluA, RSV, and Flu B in Samples in Neat STM and Detergent Formulations.** | | | | | | |
|---|---|---|---|---|---|---|
| | **100% STM or 100% Supplemented STM** | **Positive/Tests** | **Mean Ct** | **Stdev Ct** | **Ct Range** | **Mean RFU** |
| Flu A | STM + patient swab | 19/19 | 33.62 | 1 | 3.24 | 20,627 |
| | STM No patient swab | 4/4 | 34.61 | 0.23 | 0.55 | 26,787 |
| | 100% Endozime BioClean + patient swab | 0/17 | - | - | - | 86 |
| | 100% Endozime BioClean No patient swab | 4/4 | 32.56 | 0.09 | 0.22 | 30,416 |
| | 100% Endozime XP +patient swab | 0/16 | - | - | - | 41 |
| | 100% Endozime XP No patient swab | 4/4 | 32.83 | 0.4 | 0.81 | 29,410 |
| | 100% Elementum AW + patient swab | 1/15 | 38.06 | - | 0 | 969 |
| | 100% Elementum AW No patient swab | 0/4 | - | - | - | 95 |
| RSV | STM + patient swab | 19/19 | 35.4 | 0.85 | 3.33 | 12,280 |
| | STM No patient swab | 4/4 | 36.15 | 0.4 | 0.97 | 12,714 |
| | 100% Endozime BioClean + patient swab | 8/17 | 40.36 | 1.24 | 3.42 | 2,147 |
| | 100% Endozime BioClean No patient swab | 4/4 | 34.37 | 0.14 | 0.35 | 14,899 |
| | 100% Endozime XP + patient swab | 5/16 | 40.58 | 0.88 | 2.42 | 1,389 |
| | 100% Endozime XP No patient swab | 4/4 | 34.77 | 0.1 | 0.21 | 13,505 |
| | 100% Elementum AW + patient swab | 7/15 | 40.94 | 0.99 | 2.39 | 1,831 |
| | 100% Elementum AW No patient swab | 3/4 | 41.28 | 0.96 | 1.88 | 3,033 |
| Flu B | STM + patient swab | 19/19 | 35.96 | 0.96 | 3.49 | 5,561 |
| | STM No patient swab | 3/4 | 37.53 | 0.82 | 1.46 | 4,595 |
| | 100% Endozime BioClean + patient swab | 0/17 | - | - | - | 38 |
| | 100% Endozime BioClean No patient swab | 4/4 | 34.51 | 0.68 | 1.22 | 9,786 |
| | 100% Endozime XP + patient swab | 0/16 | | | | 21 |
| | 100% Endozime XP No patient swab | 4/4 | 35.08 | 0.3 | 0.66 | 8,921 |
| | 100% Elementum AW + patient swab | 0/15 | - | - | - | 40 |
| | 100% Elementum AW No patient swab | 0/4 | - | - | - | 30 |
| IC | STM + patient swab | 19/19 | 33.85 | 0.69 | 2.39 | 5,400 |
| | STM No patient swab | 4/4 | 34.84 | 0.18 | 0.42 | 5,216 |
| | 100% Endozime BioClean + patient swab | 17/17 | 33.87 | 0.99 | 4.41 | 5,946 |
| | 100% Endozime BioClean No patient swab | 4/4 | 33.37 | 0.22 | 0.49 | 6,082 |
| | 100% Endozyme XP + patient swab | 16/16 | 33.57 | 0.62 | 1.94 | 6,273 |
| | 100% Endozyme XP No patient swab | 4/4 | 33.31 | 0.31 | 0.7 | 5,634 |
| | 100% Elementum AW + patient swab | 15/15 | 33.78 | 1.02 | 3.24 | 6,190 |
| | 100% Elementum AW No patient swab | 4/4 | 33.62 | 0.19 | 0.4 | 5,936 |

| **Table 12. Detection of FluA, RSV, and Flu B, in Samples in Formulations of STM or Detergents Diluted in STM.** | | | | | | |
|---|---|---|---|---|---|---|
| | **100% STM or Diluted STM Formulations** | **Positive/Test s** | **Mean Ct** | **Stdev Ct** | **Ct Range** | **Mean RFU** |
| Flu A | STM + patient swab | 19/19 | 33.62 | 1 | 3.24 | 20,627 |
| | STM No patient swab | 4/4 | 34.61 | 0.23 | 0.55 | 26,787 |
| | 65% Endozime BioClean + patient swab | 18/18 | 33.51 | 1.5 | 5.6 | 21,194 |
| | 65% Endozime BioClean No patient swab | 4/4 | 32.6 | 0.33 | 0.74 | 32,303 |
| | 65% Endozime XP + patient swab | 23/23 | 33.13 | 0.34 | 1.34 | 20,789 |
| | 65% Endozime XP No patient swab | 4/4 | 32.71 | 0.19 | 0.43 | 30,365 |
| | 65% Elementum AW + patient swab | 0/18 | - | - | - | 71 |
| | 65% Elementum AW No patient swab | 4/4 | 34.11 | 0.28 | 0.66 | 26,688 |
| RSV | STM + patient swab | 19/19 | 35.4 | 0.85 | 3.33 | 12,280 |
| | STM No patient swab | 4/4 | 36.15 | 0.4 | 0.97 | 12,714 |
| | 65% Endozime BioClean + patient swab | 18/18 | 35.98 | 2.5 | 8.34 | 11,230 |
| | 65% Endozime BioClean No patient swab | 4/4 | 34.57 | 0.27 | 0.59 | 13,789 |
| | 65% Endozime XP + patient swab | 23/23 | 35.21 | 0.54 | 2.37 | 11,906 |
| | 65% Endozime XP No patient swab | 4/4 | 34.66 | 0.09 | 0.2 | 14,044 |
| | 65% Elementum AW + patient swab | 6/18 | 40.77 | 0.86 | 2.36 | 1,297 |
| | 65% Elementum AW No patient swab | 4/4 | 36.24 | 0.5 | 1.05 | 11,643 |
| Flu B | STM + patient swab | 19/19 | 35.96 | 0.96 | 3.49 | 5,561 |
| | STM No patient swab | 3/4 | 37.53 | 0.82 | 1.46 | 4,595 |
| | 65% Endozime BioClean + patient swab | 18/18 | 35.94 | 1.68 | 6.19 | 5,713 |
| | 65% Endozime BioClean No patient swab | 4/4 | 34.87 | 0.4 | 0.98 | 9,305 |
| | 65% Endozime XP + patient swab | 23/23 | 35.12 | 0.58 | 2 | 5,980 |
| | 65% Endozime XP No patient swab | 4/4 | 34.94 | 0.16 | 0.36 | 8,762 |
| | 65% Elementum AW + patient swab | 0/18 | - | - | - | 40 |
| | 65% Elementum AW No patient swab | 4/4 | 36.44 | 0.47 | 1.1 | 8,009 |
| IC | STM + patient swab | 19/19 | 33.85 | 0.69 | 2.39 | 5,400 |
| | STM No patient swab | 4/4 | 34.84 | 0.18 | 0.42 | 5,216 |
| | 65% Endozime BioClean + patient swab | 18/18 | 33.69 | 0.61 | 1.99 | 5,342 |
| | 65% Endozime BioClean No patient swab | 4/4 | 33.08 | 0.3 | 0.65 | 6,014 |
| | 65% Endozime XP + patient swab | 23/23 | 33.67 | 0.54 | 2.42 | 5,474 |
| | 65% Endozime XP No patient swab | 4/4 | 32.92 | 0.34 | 0.74 | 5,876 |
| | 65% Elementum AW + patient swab | 18/18 | 33.73 | 0.87 | 3.79 | 6,097 |
| | 65% Elementum AW No patient swab | 4/4 | 33.21 | 0.21 | 0.45 | 6,513 |

### Example 7: Lithium Lauryl Sulfate (LLS) for Recovery of Viral Nucleic Acids

Formulations containing lithium lauryl sulfate (LLS) were tested for recovering human immunodeficiency virus (HIV) nucleic acids (NA) from dried blood spots (DBS) and cytomegalovirus (CMV) NA from whole blood diluent (WBD).

Different percentages of LLS in STM and blood processing media (BPM; a whole-blood diluent which contains 6% LLS) were tested. To prepare the HIV-1 positive dried blood spots, Whatman 903 Specimen Collection Cards were spotted with 70 µL of HIV positive blood (whole blood spiked with HIV-1 to a concentration of 5x10⁴ copies/mL) and allowed to air dry for 24 hours. The dried blood spots were then punched out and transferred to an STM reagent containing one of several different LLS concentrations (0%, 1%, 3%, 5%, or 7% by weight; i.e., w/v (0-100 g/L)). These STM reagents were otherwise substantially identical to the STM formulation disclosed in Example 1, above. Following a 30-minute incubation, the STM was transferred to a separate tube and tested in replicates of 5 using the Aptima HIV-1 Quant Dx Assay (Hologic, Inc., cat. no. PRD-03565) according to manufacturer's instructions. The results from this study were as follows (average log copies/mL recovery, standard deviation) and are presented in Table 13 (Experiment 1). Based on these data, STM containing from 3% to 7% LLS (w/v) all showed robust amplification and detection results (about 3.5 to 3.7 log copy/mL recovery), while 0% and 1% showed lower recovery. In another experiment, using 10% LLS (w/v) also showed lower recovery (not shown).

In a further study, HIV-1 was recovered from DBS in STM containing 0%, 1%, 1.5%, 2%, 2.5%, or 3% LLS (w/v). Dried blood spot samples were prepared and recovered as is described above. Following a 30-minute incubation in an STM formulations substantially identical to the formulation in Example 1, except that LLS was present in the STM at 0, 10, 15, 20, 25, or 30 g/L, the samples were tested for the presence of HIV-1 nucleic acids using the Aptima HIV-1 assay (cat. no. PRD-03565). The results of this study are presented in Table 13 (Experiment 2) and showed a loss of assay performance below 1.5% LLS. Thus, a preferred LLS concentration is above 1.5% (w/v). These data show that sample collection media formulations containing from about 1.5% to about 7% LLS provide robust nucleic acid and recovery. The highest concentrations were obtained at about 2% to about 5% LLS.

| **Table 13: LLS Concentration for Maximum Recovery of HIV-1 Nucleic Acids from Dried Blood Spots (DBS) Specimens** | | | | |
|---|---|---|---|---|
| Experiment | LLS concentration (%) | Mean | Std Dev | Observations |
| Experiment 1: STM with 1-7% (w/v) LLS | 0.0 | 3.45 | 0.21 | 5 |
| | 1.0 | 3.25 | 0.32 | 5 |
| | 3.0 | 3.69 | 0.11 | 5 |
| | 5.0 | 3.73 | 0.14 | 5 |
| | 7.0 | 3.33 | 0.14 | 5 |
| Experiment 2: STM with 1-3% (w/v) LLS | 0.0 | 3.39 | 0.15 | 25 |
| | 1.0 | 3.36 | 0.19 | 25 |
| | 1.5 | 3.32 | 0.14 | 25 |
| | 2.0 | 3.60 | 0.12 | 25 |
| | 2.5 | 3.69 | 0.13 | 25 |
| | 3.0 | 3.72 | 0.12 | 25 |

### Example 8: Protease Stability in E-STM at Room Temperature

This example discusses changes in formulation and how those changes affect formulation stability at various temperatures, which in turn affects protease stability at those temperatures. The protease present in the formulations in the example is subtilisin. Three formulations were stored at room temperature, 4°C, or -20°C for two weeks and aspiration/dispense experiments were carried out as described in Example 1. The formulations were STM, 65% Endozime BioClean in STM, and 100% Endozime BioClean supplemented with LLS to a final concentration of 3% (w/v). While samples in STM had unresolved clots as expected, the 65% Endozime BioClean formulation lost the ability to resolve clots (Table 14). The 100% EndoZime BioClean with 3% (w/w) LLS formulation, in contrast, did have the ability to resolve clots.

| **Table 14. Aspiration/Dispense Data Comparing Formulations With and Without LLS.** | | | |
|---|---|---|---|
| **Formulation** | **Aspiration/Dispense Result After Storage at the Indicated Temperatures** | | |
| | **Room temperature** | **4°C** | **-20°C** |
| **STM** | High clot rate | High clot rate | High clot rate |
| **65% EndoZime BioClean in STM** | High clot rate | No clots | No clots |
| **100% EndoZime BioClean with 3% (w/w) LLS** | No clots | No clots | No clots |

Thus, the 65% EndoZime BioClean E-STM was considered to lack protease stability sufficient for clot prevention after two weeks at room temperature. It was noted that the chelators EDTA and EGTA were present in the 65% EndoZime BioClean E-STM because the STM contained EDTA and EGTA. The lack of protease stability was attributed to inhibition of protease in EndoZime BioClean by EDTA and EGTA. The 100% EndoZime BioClean with 3% (w/w) LLS formulation, with which clots did not occur, did not contain chelators.

Aspiration/dispense experiments confirmed that the presence of active protease plays an important role in resolving sample clots. The protease is thought to solubilize proteins in the mucoidal respiratory samples, thereby reducing aspiration pressure and reducing clots. As shown in Table 15, the presence of 3% v/v protease resolved clots in the samples. Further, the aspiration and dispense pressures with E-STM were only slightly lower than STM pressures despite resolving more clots than STM.

| **Table 15. Aspiration/Dispense Data Comparing Formulations With and Without Protease.** | | | |
|---|---|---|---|
| **Formulation** | **Aspiration/Dispense Result** | | |
| | Load #1 | Load #2 | Load #3 |
| STM | 12/18 had clots | 10/18 had clots | After 15-second vortex each, 0/10 had clots |
| 65% EndoZime BioClean with 3% w/w LLS and 2.1% w/v protease | No clots | No clots | No clots |
| 65% EndoZime BioClean with 3% w/w LLS without protease | 13/18 had clots | 13/18 had clots | After 15-second vortex each, 1/13 still had clots |

Based on the observations noted in Examples 5-7, the formulations shown in Table 16 were considered for future tests.

| **Table 16. Enhanced STM (E-STM) Formulations.** | | | |
|---|---|---|---|
| **Name** | **% Endozime Bioclean buffer** | **% v/v Protease concentration** | **% w/v Lithium lauryl sulfate (LLS) concentration** |
| E-STM 1A-P2 | 70% | 2.3 | 1% |
| E-STM 3A-P2 | 70% | 2.3 | 3% |
| E-STM 3A-P3 | 70% | 3 | 3% |
| E-STM 3A-P0 | 70% | 0 | 3% |
| E-STM 1C-P3 | 100% | 3 | 1% |
| E-STM 3C-P3 | 100% | 3 | 3% |

Further aspiration/dispense experiments showed that higher concentrations of protease were more effective at solving clots (Table 17).

| **Table 17. Aspiration/Dispense Data Comparing Formulations with Different Protease Concentrations.** | |
|---|---|
| **Formulation** | **Aspiration/Dispense Result** |
| STM | 8/20 clots |
| E-STM 1A-P2 | No clots |
| E-STM 3A-P2 | After 15 second vortexing, 1/20 clots |
| E-STM 3A-P3 | No clots |

### Example 9: Guard Band Study for Protease in E-STM

The above example illustrates that the formulations containing protease resolve clots better than those that do not contain protease. A guard band study was carried out to determine acceptable ranges of percent protease in an E-STM formulation without negative impact on aspiration and dispense pressures. E-STM formulations containing 1%, 2%, 3%, 4%, 4.5%, or 6% (w/v) protease (Savinase^{®} 12T, Strem Chemicals, Inc., Newburyport, MA, cat. no. 06-3137, 16 anson units/g) were tested and compared for performance in resolving mucoid in a sample. All formulations tested in this example contained 3% (w/v) of each of HEPES, propylene glycol, sodium xylene sulfonate, and lithium lauryl sulfate; 0.14% (w/v) of a partially fluorinated alkyl polyether, 0.1% (w/v) of a c12-c14-secondary, ethoxylated alcohol; and 0.28% (w/v) of an anionic low foam surfactant and of 1,2-benzisothiazol-3 (2H)-one. The formulations further contained protease at the following concentrations (all w/v): 0% (formulation 3G0P), 1.2% (formulation 3G1P), 2.1% (formulation 3G2P); 3% (formulation 3G3P); 3.9% (formulation 3G4P); 4.5% (formulation 3G5P); and 6% (formulation 3G6P). A commercially available specimen transport media was also included in this example (Hologic, Inc., cat. no. PRD-04423). Nasal swab samples were collected from healthy donors and the swabs were transferred to 2.9 mL of one of the test formulations or the commercial STM. Each condition was prepared in a replicate of 20. Aspiration/dispense experiments were carried out as described in Example 1. Aspiration/dispense data (Table 18) show that formulations containing from 1.2% to 6% (w/v) of a protease remove mucoid from all 20 nasal specimen while commercial STM and the formulation without protease removed mucoid in 50% to 60% of the specimen (10 tested per condition). This example showed that the formulations containing protease more efficiently resolved clotting errors and improved specimen pipetting errors.

| **Table 18: Aspiration/Dispense Data Guard Band Study for Protease.** | | | | | | | | |
|---|---|---|---|---|---|---|---|---|
| **Formulation** | **STM** | **3G0P** | **3G1P** | **3G2P** | **3G3P** | **3G4P** | **3G5P** | **3G6P** |
| **Observation from Aspiration & Dispense** | 5/10 clots (50%) | 4/10 clots (40%) | 0/10 clots | 0/10 clots | 0/10 clots | 0/10 clots | 0/10 clots | 0/10 clots |

A further study was performed to determine whether formulations having higher concentrations of protease have improved aspiration quality over formulations with lower concentrations of protease. Aspiration quality was assessed for nasal swab samples collected into one of several formulations. The aspiration quality was assessed by measuring flow rate of the collected sample/formulation through a disposable tip. In this study, formulations 3G3P, 3G6P and a formulation with 10% (w/v) protease were compared for aspiration quality. The formulation containing 10% protease was prepared as described directly above except that 10% (w/v) protease was included in the formulation (3G10P). Nasal swab samples were collected from healthy donors and the swabs were transferred to 2.9 mL of one of the test formulations. The 3G3P condition was prepared in 285 replicates, while the 3G6P and 3G10P conditions were each prepared in 230 replicates. Sample aspiration pressure data showed 1.3% clots for 3G3P conditions, 0.4% clots for 3G6P conditions, and 1.3% clots for 3G10P conditions. In this test the 3G3P condition had a higher viscosity than did either of the 3G6P and 3G10P conditions , meaning that these latter two conditions had a better aspiration quality than did the 3G3P condition. Data from this study show improved aspiration pressure profiles for formulations containing higher concentrations of protease, but that there was no difference in quality above 6% (w/v) protease in these formulations.

### Example 10: Guard Band Study for Lithium Lauryl Sulfate (LLS) in E-STM

Lithium lauryl sulfate (LLS) in commercial specimen transport media stabilizes nucleic acids for subsequent processing. A guard band study was carried out to determine acceptable ranges of percent LLS in the enhances specimen transport media formulations without negative impact on performance. E-STM formulations containing 1%, 3% or 4% (w/v) LLS were tested and compared. Several formulations were prepared as is generally described in Example 9 but without HEPES, with the addition of trisodium citrate dihydrate and protease (3% (w/v)), and with an LLS concentration of one of 1% w/v LLS (formulation 3C1L), 3% w/v LLS (formulation 3C3L), or 4% w/v LLS (formulation 3C4L). Additional formulations were prepared as generally described in Example 9 with the addition of 6% (w/v) protease and the LLS concentration was one of 2% w/v LLS (formulation 3G6P2L), 3% w/v LLS (formulation 3G6P3L), or 4% w/v LLS (formulation 3G6P4L). A commercially available specimen transport media (Hologic, Inc.) was also included.

Testing with formulations 3C1L, 3C3L, and 3C4L was performed on day 0 and after 6 days of storage at 30°C. For these tests, a nasal swab was collected for each formulation condition from 15 healthy volunteers. Each nasal swab was collected into 2.9 mL of a formulation condition. The formulations were spiked with virus and bacteria for testing with the Panther Fusion Bordetella assay (Bordetella), the Panther Fusion adenovirus/metapneumovirus/rhinovirus assay (AMR), and the Panther Fusion Flu A/B/RSV assay to a concentration equal to five times the limit of detection reported for these assays (Hologic, Inc., cat. nos. PRD-04868, PRD-04330, and PRD-04328, respectively. These spiked formulations were each divided into two incubation conditions, a 0-day incubation condition and a 6-day at 30°C incubation condition. Following the 0-day or 6-day incubation, the spiked formulations were loaded onto a Hologic Panther instrument without additional mixing and tested according to each assay protocol.

As shown in Tables 19-21 below, no difference in Ct values were noted for the 2 time points on detecting Bordetella (Table 19) or on detecting 6 viral targets (Table 20 & 21) Thus, the viral and bacterial nucleic acids were stable during 6 days of storage at 30°C in each of the formulations tested.

| **Table 19: Detection of Bordetella (Ct).** | | | | | |
|---|---|---|---|---|---|
| **Formulation** | **Test Organism** | **Day 0 Avg Ct** | **Day 0 Stdev** | **Day 6 Avg Ct** | **Day 6 Stdev** |
| 3C3L | Bordetella | 35.34 | 0.42 | 35.46 | 0.66 |
| 3C1L | Bordetella | 35.61 | 0.74 | 35.73 | 1.10 |
| 3C4L | Bordetella | 35.39 | 0.31 | 35.65 | 0.63 |
| 3C3L | Internal Control | 29.59 | 0.52 | 29.72 | 0.70 |
| 3C1L | Internal Control | 30.06 | 1.03 | 30.01 | 0.95 |
| 3C4L | Internal Control | 29.71 | 0.63 | 29.77 | 0.70 |

| **Table 20: Detection of Adenovirus, Human Metapneumovirus (hMPV), and Rhinovirus (Ct).** | | | | | |
|---|---|---|---|---|---|
| **Formulation** | **Test Organism** | **Day 0 Avg Ct** | **Day 0 Stdev** | **Day 6 Avg Ct** | **Day 6 Stdev** |
| 3C3L | Adenovirus | 34.95 | 0.24 | 35.03 | 0.39 |
| 3C1L | Adenovirus | 34.98 | 0.35 | 35.01 | 0.37 |
| 3C4L | Adenovirus | 35.13 | 0.31 | 35.06 | 0.31 |
| 3C3L | hMPV | 34.26 | 0.33 | 34.40 | 0.36 |
| 3C1L | hMPV | 34.99 | 0.56 | 34.94 | 0.53 |
| 3C4L | hMPV | 34.54 | 0.35 | 34.61 | 0.40 |
| 3C3L | Rhinovirus | 32.15 | 0.50 | 32.36 | 0.82 |
| 3C1L | Rhinovirus | 32.15 | 0.43 | 31.95 | 0.69 |
| 3C4L | Rhinovirus | 32.23 | 0.51 | 32.33 | 0.44 |
| 3C3L | Internal Control | 30.27 | 0.34 | 30.45 | 0.54 |
| 3C1L | Internal Control | 30.46 | 0.35 | 30.27 | 0.39 |
| 3C4L | Internal Control | 30.38 | 0.28 | 30.40 | 0.26 |

| **Table 21: Detection of Influenza A Virus (Flu A), Influenza B Virus (Flu B), and Respiratory Syncytial Virus (RSV).** | | | | | |
|---|---|---|---|---|---|
| **Formulation** | **Test Organism** | **Day 0 Avg Ct** | **Day 0 Stdev** | **Day 6 Avg Ct** | **Day 6 Stdev** |
| 3C3L | Flu A | 33.11 | 0.20 | 33.29 | 0.46 |
| 3C1L | Flu A | 33.10 | 0.63 | 33.38 | 0.36 |
| 3C4L | Flu A | 32.87 | 0.31 | 32.85 | 0.38 |
| 3C3L | Flu B | 34.85 | 0.34 | 34.87 | 0.29 |
| 3C1L | Flu B | 35.01 | 0.43 | 35.19 | 0.50 |
| 3C4L | Flu B | 35.14 | 0.47 | 35.08 | 0.33 |
| 3C3L | RSV | 35.73 | 0.43 | 35.87 | 0.49 |
| 3C1L | RSV | 35.89 | 0.86 | 35.80 | 0.49 |
| 3C4L | RSV | 34.61 | 0.46 | 34.57 | 0.41 |
| 3C3L | Internal Control | 33.06 | 0.42 | 32.95 | 0.32 |
| 3C1L | Internal Control | 32.90 | 0.33 | 32.94 | 0.26 |
| 3C4L | Internal Control | 33.07 | 0.34 | 32.91 | 0.47 |

Testing with formulations 3G6P2L, 3G6P3L, and 3G6P4L was performed on nasal swab sampled from healthy volunteers. Thirty (30) volunteers each provided 4 nasal swab samples which were collected into 2.9 mL of 3G6P2L, 3G6P3L, 3G6P4L, or commercial STM to provide 30 replicates of each condition. The samples in media were rocked for 15 minutes. Twenty-five (25) of the replicates for each condition were spiked with a SARS-CoV-2 virus for testing with the Aptima SARS-CoV-2 assay 3x LoD of the virus according to the package insert (Hologic, Inc., cat. no. PRD-06419), and 5 of the replicates served as negative control. Samples were loaded onto a Panther System (Hologic, Inc.) and were assayed according to instructions. The STM control condition resulted in a single clot (1/25). Without being bound by any theory, it is believed that the mixing step liquified mucoid material that may have been present in these STM conditions, giving them an improved clot profile. All conditions showed 25/25 positive results for the spiked conditions and 0/5 positive results for the negative condition. All tested conditions showed comparable average cycle time values.

Nucleic acids were stabilized for at least 6 days in these sample transport formulations containing from 1% to 6% LLS.

### Example 11: Pathogen Inactivation

The purpose of this example was to test several formulations for its ability to inactivate bacterial pathogens. Stock suspensions of *Staphylococcus aureus* and *Bordetella pertussis* were combined with either one of the formulations or with phosphate buffered saline, serially diluted, and then plated on blood agar plates and assessed for formation of colonies. The stock *Staphylococcus aureus* had a concentration of 2.6E9 CFU/mL, and the stock *Bordetella pertussis* suspension had a concentration of 4.7E9 CFU/mL. Seven (7) different test formulations were prepared as is generally described above (formulations 1A, 3A, 3C, 3D, 3E, 3G, & 3I). The formulations contained the following: lithium lauryl sulfate at 1% (w/v) (formulation 1A) or 3% (w/v), 0% or 0.1% (w/v) trisodium salt of methyl glycine diacetic acid (Trilon M, Na₃MGDA, BASF) (formulation 3E), 0% or 3% (w/v) HEPES free acid (formulations 3G & 3I), each of propylene glycol and sodium xylene sulfonate at 2% (w/v) (formulations 1A, 3A, & 3I) or 3% (w/v), 0.1% (w/v) of partially fluorinated alkyl polyether and c12-14 secondary, ethoxylated alcohol, Each of an anionic low-form surfactant and 1,2-benzisothiazol-3(2H)-one at 0.2% (w/v) (formulations 1A, 3A, & 31) or 0.3% (w/v), 2% (w/v) trisodium citrate (formulations 1A & 3A) or 3% (w/v) trisodium citrate (formulation 3C), and 2% (w/v) protease or 3% (w/v) protease (formulations 3C, 3D, 3E, & 3G). The test formulations were compared against the ability of a commercial specimen transport media to inactivate these pathogens (Hologic, Inc., San Diego, CA, cat. no. PRD-04423).

Organism control sample dilutions were prepared by combining 50 µL of a stock organism in 950 µL of PBS down to a dilution of 1E-6. Buffer control dilutions were prepared by making an initial solution containing 50 µL of a PBS in 950 µL of a formulation, and then making subsequent 50:950 µL dilutions in PBS down to a dilution of 1E-5. Test conditions were prepared separately for each organism by combining 50 µL of a stock organism in 950 µL of a formulation. Each test condition was then divided into four aliquots of 250 µL and the aliquots incubated for 15 minutes, 1 hour, 2 hours, or 4 hours. At each incubation timepoint, the corresponding test conditions serially diluted to a dilution of 1E-6 of stock. Dilutions 1E-4 to 1E-6 for organisms and 1E-5 to 1E-6 for controls were plated at 100 µL per dilution in duplicate on blood agar plates (Hardy Diagnostics, Santa Maria, CA, cat no. A10, and BD Bioscience, Franklin Lakes, NJ, cat. no BBL297876). The plates were incubated for 24 hours at 37°C. Following the 24-hour incubation, the number of colonies on each of the test condition plates and organism control plates were counted. The control dilution series show the preferred dilution of stock for growing these organisms. The buffer control series show that the buffer is not preventing the organism from growing on a plate at any given dilution. The test condition series show whether a formulation inactivates the organism.

CFU count on each plate was back calculated to the stock suspension (undiluted) to determine CFU/mL. The back calculated CFU/mL was compared to the original stock suspension and a fold reduction in pathogen under test condition was determined. Results are shown in Table 22, below.

**Table 22: Fold Reduction of Bacterial Pathogens in Sample Transport Media Formulations**

| **Formulation** | ***Staphylococcus aureus* Fold Reduction to Stock*** | ***Bordetella pertussis* Fold Reduction to Stock*** |
|---|---|---|
| STM: 15 minutes | 3.50E+05 | 2.87E+06 |
| STM: 1 hour | 2.80E+05 and >6.40E+05 | 2.87E+06 and 3.6E+06 |
| STM: 2 hour | 1.34E+06 and > 6.40E+05 | 3.6E+06 |
| STM: 4 hour | 1.34E+06 and 6.40E+05 | 3.6E+06 |
| Formulation 1A: 15 minutes | 2,274 and 2,860 | 1.44E+06 |
| Formulation 1A: 1 hour | 1.28E4 and 9.85E3 | 9.57E+05 |
| Formulation 1A: 2 hour | 2.23E+04 | Not calculated |
| Formulation 1A: 4 hour | 1.34E+05 | Not calculated |
| Formulation 3A: 15 minutes | 1.41E+04 and 2.12E+04 | 2.87E+06 |
| Formulation 3A: 1 hour | 1.27E+05 and 7.88E+04 | 2.87E+06 |
| Formulation 3A: 2 hour | 2.23E+05 | Not calculated |
| Formulation 3A: 4 hour | 1.34E+06 | Not calculated |
| Formulation 3C: 15 minutes | Not calculated | Not calculated |
| Formulation 3C: 1 hour | 8.00E+04 | >3.6E+06 |
| Formulation 3C: 2 hour | 6.40E+05 | 3.60E+06 |
| Formulation 3C: 4 hour | > 6.40E+05 | 3.60E+06 |
| Formulation 3D: 1 hour | 5.72E+04 | 1.86E+06 |
| Formulation 3D: 4 hour | 3.58E+05 | >3.7E+06 |
| Formulation 3E: 1 hour | 8.94E+04 | 1.24E+06 |
| Formulation 3E: 4 hour | 1.43E+06 | >3.7E+06 |
| Formulation 3G: 1 hour | 3.04E+04 | 3.71E+05 |
| Formulation 3G: 4 hour | 7.15E+05 | 1.86E+06 |
| Formulation 3I: 1 hour | 3.86E+04 | 3.09E+05 |
| Formulation 3I: 4 hour | 2.04E+05 | 9.28E+05 |

| | | |
|---|---|---|
| * Conditions showing two results were tested in duplicate. | | |

In Table 22, the higher values represent a greater effectiveness at target inactivation. Formulations containing 3% (w/v) LLS were more effective at inactivating target pathogens than were the formulations containing less than 3% (w/v) LLS. Formulation 3C inactivated the test pathogens as effectively as did commercial sample transport media. Based on data from these studies, a sample transport media with LLS concentration above 1% (w/v) is preferred for efficient bacterial pathogen inactivation.

A further experiment was performed for inactivation of viral pathogens. The test formulation that was used in this experiment was based on one of the better performing formulations from the bacterial inactivation experiment. The test formulation contained 3% (w/v) of each of lithium lauryl sulfate, trisodium citrate, propylene glycol, and sodium xylene sulfonate; 0.1% (w/v) of partially fluorinated alkyl polyether and of c12-c14 ethoxylated secondary alcohols; and 0.3% (w/v) of an anionic, low foam surfactant and of benzisothiazolone. The protease component was removed from the test formulation because protease activity on tissue culture would result in an inaccurate assessment of the formulation. The test formulation was compared against a commercial specimen transport media (Hologic, Inc., San Diego, CA, cat. no. PRD-04423). The test formula and the STM were each combined with stock titers of coronavirus 229E and Adenovirus serotype 2 and incubated for 1-hour at 37°C. Following the incubation, the test conditions and STM condition were diluted down to a pre-determined concentration shown to infect the tissue culture cells, and each of the dilutions were run across detergent removal columns (Pierce Biotech, Rockform, IL, cat. no. 87776) according to manufacturer's instructions. Detergent-free dilutions were added (100 µL) to separate cultures of MRC-5 cells (ATCC, Manassas, VA, cat. no. CCL-171) that had been grown to 80% to 90 % confluence. Positive control conditions were prepared using the stock viruses diluted in a cell growth media and added to the MRC-5 tissue cultures. No virus controls were also prepared to measure whether a formulation dilution condition would negatively affect the tissue cultures. Tissue cultures for all conditions were then incubated for 2 hours at 37°C to allow any virus from the test, commercial STM, or positive control conditions to infect the cells. Media was then changed to a standard cell growth media, and the cultures were further incubated. Each tissue culture was then viewed under a microscope to determine the presence and degree of cytopathic effects in the culture, and in turn determine the ability of a test formulation to inactivate viruses compared to commercial STM. The positive control cultures indicated typical cytopathic effects of each virus at each dilution. Results show that the test formulation inactivated virus as effectively as did the commercial STM.

All formulations tested in this example were effective at inactivating various pathogen, with formulations containing more than 2% (w/v) protease being more effective thank those containing 2% (w/v) protease.

### Example 12: Precipitation Studies

Precipitate formation was observed for formulation 3C, described above, when stored at 4°C. At least a 1-hour a room temperature incubation was required to get the precipitant back into solution, which is an additional step to perform before the formulations could be used. Seven formulations, including formulation 3C, were prepared and evaluated for the formation of a precipitant. The formulations included formulations 3C, 3D, 3E, 3G, and 3I (described in Example 11, above) and 3J. Formulation 3J contained 2% (w/v) propylene glycol and sodium xylene sulfonate, 3% (w/v) protease and lithium lauryl sulfonate, 0.2 % (w/v) BIT preservative and anionic low-foam surfactant, and 0.1% (w/v) of partially fluorinated alkyl polyether and c12-c14 secondary ethoxylated alcohols. Each formulation was divided into the following two conditions: -20°C storage and 4°C storage. None of formulations 3D, 3E, 3G, 3I, or 3J formed precipitates when stored at 4°C. However, formulations 3D and 3E formed precipitates when thawed from -20°C.

The formulation containing 3% (w/v) tri sodium citrate formed a precipitate when stored at 4°C. Removing that reagent from the formulation resulted in several formulations that did not form precipitates at 4°C, however some of these formulations formed precipitate when thawed from -20°C. The addition of HEPES free acid and/or the reduction in concentration of other formulation components resulted in formulations that did not form precipitates when thawed form -20°C.

## Claims

1. Use of a composition comprising at least one protease and an anionic detergent, and optionally, a buffer and/or a divalent cation chelator, for collection of a biological sample for diagnostic testing,
wherein the protease is present at about 1% w/v to about 10% w/v,
wherein the anionic detergent is present at about 2% w/v to about 6% w/v, and
wherein the biological sample is a pulmonary sample selected from the group consisting of sputum, nasal mucous, nasal discharge, an oropharyngeal swab sample, bronco alveolar lavage, bronchial washings, and nasopharyngeal swab sample.

2. The use of claim 1, wherein the protease is present at about 3% w/v to about 6% w/v, about 3% w/v, or about 6% w/v.

3. The use of claim 1 or 2, wherein the anionic detergent comprises an alkyl sulfate,
optionally wherein the alkyl sulfate is a dodecyl sulfate,
optionally wherein the anionic detergent comprises a sodium salt or a lithium salt,
optionally wherein the anionic detergent comprises lithium lauryl sulfate.

4. The use of any one of claims 1 to 3, wherein the composition further comprises a detergent builder, coupling agent, or complexing agent; optionally wherein the detergent builder, coupling agent, or complexing agent comprises sodium xylene sulfonate, zeolite, diphosphate, triphosphate, phosphonate, carbonate, citrate, nitrilotriacetic acid, ethylenediaminetetraacetic acid, diethylenetriaminepentaacetic acid, alkyl- or alkenylsuccinic acid, soluble silicates, or layered silicates; further optionally wherein the layered silicates are Hoechst SKS-6.

5. The use of any one of claims 1 to 4, wherein the composition further comprises a bleaching system, optionally wherein the bleaching system comprises a source of H2O2; further optionally wherein the source of H2O2 comprises perborate, percarbonate, a peracid-forming bleach activator, or a combination thereof.

6. The use of any one of claims 1 to 5, wherein the composition further comprises a stabilizing agent; optionally wherein the stabilizing agent comprises a propylene glycol, polyol, sugar, sugar alcohol, lactic acid, boric acid, or a boric acid derivative; further optionally wherein the boric acid derivative comprises a borate ester, an aromatic borate ester, a phenylboronic acid optionally wherein the phenyl is a substituted phenyl, or 4-formylphenylboronic acid.

7. The use of any one of claims 1 to 6, wherein the composition further comprises one or more of a clay, foam booster, suds suppressor, anti-corrosion agent, soil-suspending agent, anti-soil redeposition agent, bactericide, tarnish inhibitor, or hydrotrope, optionally wherein the hydrotrope comprises urea, tosylate, cumene sulfonate or xylene sulfonate.

8. The use of any one of claims 1 to 7, wherein the composition further comprises a surfactant; optionally wherein the surfactant is present at less than about 10% by weight of the composition.

9. The use of any one of claims 1 to 8, wherein the composition has a pH of 3 to 14, optionally wherein the composition has a pH of 6 to 7, 7 to 8, 8 to 9, 9 to 10, or 10 to 11.

10. The use of any one of claims 1 to 9, wherein the buffer is a phosphate buffer, sodium phosphate, HEPES, or an alkali salt thereof.

11. The use of any one of claims 1 to 10, wherein the divalent cation chelator comprises a magnesium chelator and/or a calcium chelator, optionally wherein the magnesium chelator and/or calcium chelator comprises Trilon M, trisodium citrate, EDTA, or EGTA.

12. The use of any one of claims 1 to 11, wherein the composition further comprises a preservative; wherein the preservative comprises a microbicide, bactericide, biocide, fungicide, and/or benzisothiazolinone.

13. The use of any one of claims 1 to 12, wherein the biological sample comprises or is suspected of comprising extracellular nucleic acids of a respiratory pathogen.

14. A method of processing a biological sample, wherein the biological sample is a pulmonary sample selected from the group consisting of sputum, nasal mucous, nasal discharge, an oropharyngeal swab sample, bronco alveolar lavage, bronchial washings, and nasopharyngeal swab sample, the method comprising contacting the biological sample with a composition as defined in any one of claims 1 to 13.

15. The method of claim 14, wherein
the biological sample comprises a target nucleic acid,
the method optionally further comprises isolating the target nucleic acid from the biological sample, wherein isolating the target nucleic acid comprises binding the target nucleic acid to at least one capture oligomer, and
the method optionally further comprises amplifying the target nucleic acid.

16. A method of inactivating infectious pathogens in a biological sample, wherein the biological sample is a pulmonary sample selected from the group consisting of sputum, nasal mucous, nasal discharge, an oropharyngeal swab sample, bronco alveolar lavage, bronchial washings, and nasopharyngeal swab sample, the method comprising contacting the biological sample with a composition as defined in any one of claims 1 to 13.

## Patentansprüche

1. Verwendung einer Zusammensetzung, umfassend mindestens eine Protease und ein anionisches Reinigungsmittel, und optional einen Puffer und/oder einen Chelatbildner für zweiwertige Kationen, zum Erfassen einer biologischen Probe für Diagnosetests,
wobei die Protease mit etwa 1 Gew.-% bis etwa 10 Gew.-% vorhanden ist,
wobei das anionische Reinigungsmittel mit etwa 2 Gew.-% bis etwa 6 Gew.-% vorhanden ist, und
wobei die biologische Probe eine pulmonale Probe ist, die ausgewählt ist aus der Gruppe bestehend aus Sputum, Nasenschleim, Nasenausfluss, einer Oropharyngealabstrichprobe, bronchoalveolärer Lavage, Bronchialwaschungen und einer Nasopharyngealabstrichprobe.

2. Verwendung nach Anspruch 1, wobei die Protease mit etwa 3 Gew.-% bis etwa 6 Gew.-%, etwa 3 Gew.-% oder etwa 6 Gew.-% vorhanden ist.

3. Verwendung nach Anspruch 1 oder 2, wobei das anionische Reinigungsmittel ein Alkylsulfat umfasst,
wobei optional das Alkylsulfat ein Dodecylsulfat ist,
wobei optional das anionische Reinigungsmittel ein Natriumsalz oder ein Lithiumsalz umfasst,
wobei optional das anionische Reinigungsmittel Lithiumlaurylsulfat umfasst.

4. Verwendung nach einem der Ansprüche 1 bis 3, wobei die Zusammensetzung ferner einen Reinigungsmittelgerüststoff, ein Kopplungsmittel oder Komplexierungsmittel umfasst; wobei optional der Reinigungsmittelgerüststoff, das Kopplungsmittel oder das Komplexierungsmittel Natriumxylolsulfonat, Zeolith, Diphosphat, Triphosphat, Phosphonat, Carbonat, Citrat, Nitrilotriessigsäure, Ethylendiamintetraessigsäure, Diethylentriaminpentaessigsäure, Alkyl- oder Alkenylbernsteinsäure, lösliche Silikate oder Schichtsilikate umfasst, wobei optional die Schichtsilikate Hoechst SKS-6 sind.

5. Verwendung nach einem der Ansprüche 1 bis 4, wobei die Zusammensetzung ferner ein Bleichsystem umfasst, wobei optional das Bleichsystem eine Quelle von H2O2 umfasst; wobei ferner optional die Quelle von H2O2 Perborat, Percarbonat, einen Persäure ausbildenden Bleichaktivator oder eine Kombination daraus umfasst.

6. Verwendung nach einem der Ansprüche 1 bis 5, wobei die Zusammensetzung ferner ein Stabilisierungsmittel umfasst, wobei das Stabilisierungsmittel ein Propylenglycol, Polyol, Zucker, Zuckeralkohol, Essigsäure, Borsäure oder ein Borsäurederivat umfasst; wobei ferner optional das Borsäurederivat ein Boratester, ein aromatisches Boratester, eine Phenylboronsäure umfasst, wobei optional das Phenyl ein substituiertes Phenyl oder 4-Formylphenylboronsäure ist.

7. Verwendung nach einem der Ansprüche 1 bis 6, wobei die Zusammensetzung ferner eines oder mehrere aus einem Ton, einem Schaumverstärker, einem Seifenlaugeunterdrücker, einem Antikorrosionsmittel, einem Schmutztragemittel, einem Mittel zur Verhinderung einer erneuten Schmutzablagerung, einem Bakterizid, einem Anlaufinhibitor oder einem Hydrotrop, wobei optional das Hydrotrop Harnstoff, Tosylat, Cumolsulfonat oder Xylolsulfonat umfasst.

8. Verwendung nach einem der Ansprüche 1 bis 7, wobei die Zusammensetzung ferner ein Tensid umfasst, wobei optional das Tensid mit weniger als etwa 10 Gew.-% der Zusammensetzung vorhanden ist.

9. Verwendung nach einem der Ansprüche 1 bis 8, wobei die Zusammensetzung einen pH-Wert von 3 bis 14 aufweist, wobei optional die Zusammensetzung einen pH-Wert von 6 bis 7, 7 bis 8, 8 bis 9, 9 bis 10 oder 10 bis 11 aufweist.

10. Verwendung nach einem der Ansprüche 1 bis 9, wobei der Puffer ein Phosphatpuffer, ein Natriumpuffer, HEPES, oder ein Alkalisalz davon ist.

11. Verwendung nach einem der Ansprüche 1 bis 10, wobei der Chelatbildner für zweiwertige Kationen einen Magnesiumchelatbildner und/oder einen Calciumchelatbildner umfasst, wobei optional der Magnesiumchelatbildner und/oder der Calciumchelatbildner Trilon M, Trinatriumcirtrat, EDTA oder EGTA umfassen.

12. Verwendung nach einem der Ansprüche 1 bis 11, wobei die Zusammensetzung ferner einen Konservierungsstoff umfasst, wobei der Konservierungsstoff ein Mikrobizid, ein Bakterizid, ein Biozid, ein Fungizid und/oder Benzisothiazolinon umfasst.

13. Verwendung nach einem der Ansprüche 1 bis 12, wobei die biologische Probe extrazelluläre Nukleinsäure eines respiratorischen Erregers umfasst oder der Verdacht besteht, dass sie diese umfasst.

14. Verfahren zum Verarbeiten einer biologischen Probe, wobei die biologische Probe eine pulmonale Probe ist, die ausgewählt ist aus der Gruppe bestehend aus Sputum, Nasenschleim, Nasenausfluss, einer Oropharyngealabstrichprobe, bronchoalveolärer Lavage, Bronchialwaschungen und einer Nasopharyngealabstrichprobe, das Verfahren umfassend das Inkontaktbringen der biologischen Probe mit einer Zusammensetzung, wie sie in einem der Ansprüche 1 bis 13 definiert ist.

15. Verfahren nach Anspruch 14, wobei
die biologische Probe eine Zielnukleinsäure umfasst,
das Verfahren optional ferner das Isolieren der Zielnukleinsäure von der biologischen Probe umfasst, wobei das Isolieren der Zielnukleinsäure das Binden der Zielnukleinsäure an mindestens ein Fänger-Oligomer umfasst, und
das Verfahren optional ferner das Amplifizieren der Zielnukleinsäure umfasst.

16. Verfahren zum Inaktivieren von infektiösen Erregern in einer biologischen Probe, wobei die biologische Probe eine pulmonale Probe ist, die ausgewählt ist aus der Gruppe bestehend aus Sputum, Nasenschleim, Nasenausfluss, einer Oropharyngealabstrichprobe, bronchoalveolärer Lavage, Bronchialwaschungen und einer Nasopharyngealabstrichprobe, das Verfahren umfassend das Inkontaktbringen der biologischen Probe mit einer Zusammensetzung, wie sie in einem der Ansprüche 1 bis 13 definiert ist.

## Revendications

1. Utilisation d'une composition comprenant au moins une protéase et un détergent anionique, et éventuellement un tampon et/ou un chélateur de cations divalents, pour la collecte d'un échantillon biologique pour un test de diagnostic,
dans laquelle la protéase est présente à hauteur d'environ 1 % p/v à environ 10 % p/v,
dans laquelle le détergent anionique est présent à hauteur d'environ 2 % p/v à environ 6 % p/v, et
dans laquelle l'échantillon biologique est un échantillon pulmonaire sélectionné dans le groupe consistant en crachat, mucus nasal, écoulement nasal, échantillon oropharyngé prélevé par écouvillonnage, lavage broncho-alvéolaire, lavages bronchiaux et échantillon nasopharyngé prélevé par écouvillonnage.

2. Utilisation selon la revendication 1, dans laquelle la protéase est présente à hauteur d'environ 3 % p/v à environ 6 % p/v, d'environ 3 % p/v ou d'environ 6 % p/v.

3. Utilisation selon la revendication 1 ou 2, dans laquelle le détergent anionique comprend un sulfate d'alkyle,
éventuellement dans laquelle le sulfate d'alkyle est un sulfate de dodécyle,
éventuellement dans laquelle le détergent anionique comprend un sel de sodium ou un sel de lithium,
éventuellement dans laquelle le détergent anionique comprend du laurylsulfate de lithium.

4. Utilisation selon l'une quelconque des revendications 1 à 3, dans laquelle la composition comprend en outre un adjuvant pour détergents, un agent de couplage ou agent complexant, éventuellement dans laquelle l'adjuvant pour détergents, l'agent de couplage ou l'agent complexant comprend xylènesulfonate de sodium, zéolite, diphosphate, triphosphate, phosphonate, carbonate, citrate, acide nitrilotriacétique, acide éthylènediaminetétraacétique, acide diéthylènetriaminepentaacétique, acide alkyl- ou alcénylsuccinique, silicates solubles ou silicates stratifiés ; éventuellement en outre dans laquelle les silicates stratifiés sont du Hoechst SKS-6.

5. Utilisation selon l'une quelconque des revendications 1 à 4, dans laquelle la composition comprend en outre un système de blanchiment, éventuellement dans laquelle le système de blanchiment comprend une source de H2O2 ; éventuellement en outre dans laquelle la source de H2O2 comprend du perborate, du percarbonate, un activateur de blanchiment formant un peracide ou une combinaison de ceux-ci.

6. Utilisation selon l'une quelconque des revendications 1 à 5, dans laquelle la composition comprend en outre un agent stabilisant ; éventuellement dans laquelle l'agent stabilisant comprend un propylène glycol, un polyol, du sucre, de l'alcool de sucre, de l'acide lactique, de l'acide borique ou un dérivé d'acide borique ; éventuellement en outre dans laquelle le dérivé d'acide borique comprend un ester de borate, un ester de borate aromatique, un acide phénylboronique, éventuellement dans laquelle le phényle est un phényle substitué ou un acide 4-formylphénylboronique.

7. Utilisation selon l'une quelconque des revendications 1 à 6, dans laquelle la composition comprend en outre un ou plusieurs parmi une argile, un renforçateur de mousse, un inhibiteur de mousse, un agent anti-corrosion, un agent de mise en suspension des salissures, un agent anti-redéposition des salissures, un bactéricide, un inhibiteur de ternissement ou un hydrotrope, éventuellement dans laquelle l'hydrotrope comprend urée, tosylate, sulfonate de cumène ou sulfonate de xylène.

8. Utilisation selon l'une quelconque des revendications 1 à 7, dans laquelle la composition comprend en outre un tensioactif ; éventuellement dans laquelle le tensioactif est présent à hauteur de moins d'environ 10 % en poids de la composition.

9. Utilisation selon l'une quelconque des revendications 1 à 8, dans laquelle la composition a un pH de 3 à 14, éventuellement dans laquelle la composition a un pH de 6 à 7, 7 à 8, 8 à 9, 9 à 10 ou 10 à 11.

10. Utilisation selon l'une quelconque des revendications 1 à 9, dans laquelle le tampon est un tampon de phosphate, du phosphate de sodium, de l'HEPES ou un sel alcalin de ceux-ci.

11. Utilisation selon l'une quelconque des revendications 1 à 10, dans laquelle le chélateur de cations divalents comprend un chélateur de magnésium et/ou un chélateur de calcium, éventuellement dans laquelle le chélateur de magnésium et/ou le chélateur de calcium comprend/comprennent Trilon M, citrate trisodique, EDTA ou EGTA.

12. Utilisation selon l'une quelconque des revendications 1 à 11, dans laquelle la composition comprend en outre un conservateur ; dans laquelle le conservateur comprend un microbicide, un bactéricide, un biocide, un fongicide et/ou du benzisothiazolinone.

13. Utilisation selon l'une quelconque des revendications 1 à 12, dans laquelle l'échantillon biologique comprend ou est suspecté de comprendre des acides nucléiques extracellulaires d'un agent pathogène des voies respiratoires.

14. Procédé de traitement d'un échantillon biologique, dans lequel l'échantillon biologique est un échantillon pulmonaire sélectionné dans le groupe consistant en crachat, mucus nasal, écoulement nasal, échantillon oropharyngé prélevé par écouvillonnage, lavage broncho-alvéolaire, lavages bronchiaux et échantillon nasopharyngé prélevé par écouvillonnage, le procédé comprenant la mise en contact de l'échantillon biologique avec une composition telle que définie dans l'une quelconque des revendications 1 à 13.

15. Procédé selon la revendication 14, dans lequel
l'échantillon biologique comprend un acide nucléique cible,
le procédé comprend en outre éventuellement l'isolement de l'acide nucléique cible de l'échantillon biologique, dans lequel l'isolement de l'acide nucléique cible comprend la liaison de l'acide nucléique cible à au moins un oligomère de capture, et
le procédé comprend en outre éventuellement l'amplification de l'acide nucléique cible.

16. Procédé d'inactivation d'agents pathogènes infectieux dans un échantillon biologique, dans lequel l'échantillon biologique est un échantillon pulmonaire sélectionné dans le groupe consistant en crachat, mucus nasal, écoulement nasal, échantillon oropharyngé prélevé par écouvillonnage, lavage broncho-alvéolaire, lavages bronchiaux et échantillon nasopharyngé prélevé par écouvillonnage, le procédé comprenant la mise en contact de l'échantillon biologique avec une composition telle que définie dans l'une quelconque des revendications 1 à 13.
